# EUROPEAN PATENT SPECIFICATION

(11) **EP 3 140 410 B1**
(45) Date of publication and mention of the grant of the patent: **11.09.2019**
(21) Application number: 15730972.5
(22) Date of filing: 06.05.2015
(51) Int. Cl.: C12P 7/02, C12N 9/16, C12N 15/82, C12N 9/02, C12P 7/04

(54) **DRIMENOL SYNTHASES AND METHOD OF PRODUCING DRIMENOL**
DRIMENOLSYNTHASEN UND VERFAHREN ZUR HERSTELLUNG VON DRIMENOL
DRIMÉNOL SYNTHASES ET MÉTHODE DE PRODUCTION DE DRIMÉNOL

(30) Priority: 06.05.2014 WO PCT/CN2014/076890
(43) Date of publication of application: 15.03.2017
(73) Proprietor: Firmenich SA, 1211 Geneva 8 (CH)
(72) Inventor: HAEFLIGER, Olivier, Shanghai 201108 (CN); HE, Xiu-Feng, Shanghia 201108 (CN); ZHANG, Yu-Hua, Shanghai 201108 (CN); SCHALK, Michel, 1211 Geneva 8 (CH); DEGUERRY, Fabienne, CH-1211 Geneva 8 (CH)
(74) Representative: Schweiger, Georg
(86) International application number: PCT/EP2015/059988
(87) International publication number: WO 2015/169871

(56) References cited:
- WO-A1-2012/058636
- WO-A1-2013/058655
- Bryan Wilkinson Pyle et al.: "Molecular Cloning and Characterization of Sesquiterpene Synthases from Valeriana officinalis", UNIVERSITY OF CALGARY, Department of Biological Sciences , 1 January 2012 (2012-01-01), July 2012 (2012-07), pages 1-94, XP055211211, Retrieved from the Internet: URL:http://theses.ucalgary.ca/bitstream/11 023/129/2/ucalgary_2012_pyle_bryan.pdf [retrieved on 2015-09-04]
- MUNOZ-CONCHA ET AL: "Presence of polygodial and drimenol in Drimys populations from Chile", BIOCHEMICAL SYSTEMATICS AND ECOLOGY, PERGAMON PRESS, GB, vol. 35, no. 7, 29 April 2007 (2007-04-29) , pages 434-438, XP022052534, ISSN: 0305-1978, DOI: 10.1016/J.BSE.2006.10.019

## Description

### Technicalfield

The field relates to methods of producing drimenol, said method comprising contacting at least one polypeptide with farnesyl pyrophosphate (FPP). In particular, said method may be carried out *in vitro* or *in vivo* to produce drimenol, a very useful compound in the fields of perfumery. Also provided herein is an amino acid sequence of a polypeptide useful in the methods provided herein. A nucleic acid encoding the polypeptide of an embodiment herein and an expression vector containing said nucleic acid are also provided herein. A non-human host organism or a cell transformed to be used in the method of producing drimenol is further provided herein.

### Background

Terpenes are found in most organisms (microorganisms, animals and plants). These compounds are made up of five carbon units called isoprene units and are classified by the number of these units present in their structure. Thus monoterpenes, sesquiterpenes and diterpenes are terpenes containing 10, 15 and 20 carbon atoms respectively. Sesquiterpenes, for example, are widely found in the plant kingdom. Many sesquiterpene molecules are known for their flavor and fragrance properties and their cosmetic, medicinal and antimicrobial effects. Numerous sesquiterpene hydrocarbons and sesquiterpenoids have been identified.

Biosynthetic production of terpenes involves enzymes called terpene synthases. There is virtually an infinity of sesquiterpene synthases present in the plant kingdom, all using the same substrate (farnesyl pyrophosphate, FPP) but having different product profiles. Genes and cDNAs encoding sesquiterpene synthases have been cloned and the corresponding recombinant enzymes characterized. Methods for producing modified terpene synthases are described in WO 2012/058636.

Currently the main source for drimenol are plants naturally containing drimenol and the contents of drimenol in these natural sources are low. Drimenol concentrations have been quantified in leaves of several populations of Drimys winteri and Drimys andina (Munoz-Concha et al., Biochem. System. Ecol., 35: 434-438, (2017)). Nucleic acids encoding drimenol synthase enzymes have been isolated from plant species such as *Valeriana officinalis* and/or *Persicaria hydropiper* and have been used to synthesize drimenol *in vitro* or in genetically modified host cells or organisms (WO 2013/058655; Pyle ("Molecular Cloning and Characterization of Sesquiterpene Synthases from Valeriana officinalis", University of Calgary, Thesis, (2012)). Chemical synthesis approaches have been developed but are still complex and not cost-effective.

### Summary

Provided herein is a method of producing drimenol comprising:
i) contacting farnesyl diphosphate (FPP) with a polypeptide having drimenol synthase activity and having at least 70% sequence identity to a sequence of SEQ ID NO: 2 to produce the drimenol; and
ii) optionally isolating the drimenol.

Further provided herein is an isolated polypeptide having drimenol synthase activity comprising an amino acid sequence having at least 70% sequence identity to an amino acid sequence of SEQ ID NO: 2.

Also provided herein is an isolated nucleic acid molecule encoding a polypeptide having at least 70% sequence identity to a sequence of SEQ ID NO: 2.

Also provided is a vector comprising the above mentioned nucleic acid molecule and a non-human host organism or cell comprising the above mentioned nucleic acid molecule or vector.

### Description of the drawings

**Figure 1****.** GCMS analysis of the leaves of *Drimys lanceolata* and *Drimys winteri.*
**Figure 2****.** GCMS analysis of the sesquiterpenes produced by the recombinant DlTps589 in *in-vitro* assays. A. Total ion chromatogram of the sesquiterpene profile of an incubation of the recombinant DlTps589 protein with FPP. B. Negative control performed in the same conditions with *E. coli* cells transformed with an empty plasmid. C. Mass spectrum of the peak at 11.76 min. D. Mass spectrum of an authentic standard of (-)-drimenol.
**Figure 3****.** GCMS analysis of the sesquiterpenes produced *in vivo* by the recombinant DlTps589 in engineered bacteria cells. A. Total ion chromatogram. B. Mass spectrum of the peak at 11.49 min. C. Mass spectrum of an authentic standard of (-)-drimenol. The compound eluting at 10.98 min is farnesol produced by the DlTps589 enzyme or resulting from the hydrolysis of excess FPP produced by the *E. coli* cells.
**Figure 4****.** Structure of (-)-drimenol produced by the recombinant DlTps589 synthase.
**Figure 5****.** Chiral GC\FID chromatograms of *(-)*-drimenol produced by the recombinant enzyme (upper), racemic drimenol obtained chemically (middle) and authentic *(-)*-drimenol (lower).
**Figure 6****.** Total ion chromatogram of GCMS analysis of the sesquiterpenes produced in *in-vitro* assays by the recombinant proteins SCH51-3228-9 (A), SCH51-998-28 (B) or SCH52-13163-6 (C).
**Figure 7****.** Total ion chromatogram of GCMS analysis of the sesquiterpenes produced *in vivo* by engineered bacteria cells expressing the different recombinant proteins SCH51-3228-9 (A), SCH51-998-28 (B) or SCH52-13163-6 (C). The farnesol detected results from the hydrolysis of excess FPP produced by the *E. coli* cells or could be in part produced by the recombinant proteins.

### Detailed Description

For the descriptions herein and the appended claims, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of".

In one aspect, provided here is a method of producing drimenol comprising:
i) contacting farnesyl diphospate (FPP) with a polypeptide having drimenol synthase activity and comprising an amino acid sequence having at least 70%, particularly 75%, particularly 80%, particularly 85%, particularly 90%, particularly 95%, particularly 96%, particularly 97%, particularly 98% or particularly 99% or more sequence identity to a sequence of SEQ ID NO: 2 to produce the drimenol; and
ii) optionally isolating the drimenol.

In one aspect, the drimenol is isolated.

Further provided herein is an isolated polypeptide having drimenol synthase activity comprising an amino acid sequence having at least 70%, particularly 75%, particularly 80%, particularly 85%, particularly 90%, particularly 95%, particularly 96%, particularly 97%, particularly 98% or more particularly 99% or more identity to amino acid sequence of SEQ ID NO: 2.

Further provided herein is an isolated nucleic acid molecule encoding a polypeptide comprising an amino acid sequence having at least 70%, particularly 75%, particularly 80%, particularly 85%, particularly 90%, particularly 95%, particularly 96%, particularly 97%, particularly 98% or more particularly 99% or more identity to amino acid sequence of SEQ ID NO: 2 .

Further provided herein is a nucleic acid molecule comprising the sequence selected from the group consisting of SEQ ID NO:1 and SEQ ID NO: 3. Further disclosed herein are nucleic acid molecules comprising the sequence from the group consisting of SEQ ID NO: 4, SEQ ID NO: 6 SEQ ID NO: 7, SEQ ID NO: 9, SEQ ID NO: 10, SEQ ID NO: 12, SEQ ID No: 13 and SEQ ID NO: 15.

Further provided herein is a method as recited in claim 1 comprising the steps of transforming a host cell or non-human organism with a nucleic acid encoding a polypeptide having at least 70%, particularly 75%, particularly 80%, particularly 85%. particularly 90%, particularly 95%, particularly 96%, particularly 97%, particularly 98% or particularly 99% or more sequence identity of the sequence of SEQ ID NO: 2 and culturing the host cell or organism under conditions that allow for the production of the polypeptide.

Further provided is at least one vector comprising the nucleic acid molecules described.

Further provided herein is a vector selected from the group of a prokaryotic vector, viral vector and a eukaryotic vector.

Further provided here is a vector that is an expression vector.

As a "drimenol synthase" or as a "polypeptide having a drimenol synthase activity", we mean here a polypeptide capable of catalyzing the synthesis of drimenol, in the form of any of its stereoisomers or a mixture thereof, starting from farnesyl diphosphate (FPP). Drimenol may be the only product or may be part of a mixture of sesquiterpenes.

The ability of a polypeptide to catalyze the synthesis of a particular sesquiterpene (for example drimenol) can be simply confirmed by performing the enzyme assay as detailed in Examples 2 to 5.

As intended herein below, "a nucleotide sequence obtained by modifying SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof' encompasses any sequence that has been obtained by changing the sequence of SEQ ID NO: 1, SEQ ID NO: 3, SEQ ID NO: 4, SEQ ID NO: 6, SEQ ID NO: 7, SEQ ID NO: 9, SEQ ID NO: 10 or SEQ ID NO: 12 or of the complement thereof using any method known in the art, for example by introducing any type of mutations such as deletion, insertion or substitution mutations. Examples of such methods are cited in the part of the description relative to the variant polypeptides and the methods to prepare them.

The percentage of identity between two peptidic or nucleotidic sequences is a function of the number of amino acids or nucleotide residues that are identical in the two sequences when an alignment of these two sequences has been generated. Identical residues are defined as residues that are the same in the two sequences in a given position of the alignment. The percentage of sequence identity, as used herein, is calculated from the optimal alignment by taking the number of residues identical between two sequences dividing it by the total number of residues in the shortest sequence and multiplying by 100.
The optimal alignment is the alignment in which the percentage of identity is the highest possible. Gaps may be introduced into one or both sequences in one or more positions of the alignment to obtain the optimal alignment. These gaps are then taken into account as non-identical residues for the calculation of the percentage of sequence identity. Alignment for the purpose of determining the percentage of amino acid or nucleic acid sequence identity can be achieved in various ways using computer programs and for instance publicly available computer programs available on the world wide web. Preferably, the BLAST program (Tatiana et al, FEMS Microbiol Lett., 1999, 174:247-250, 1999) set to the default parameters, available from the National Center for Biotechnology Information (NCBI) at http://www.ncbi.nlm.nih.gov/BLAST/bl2seq/wblast2.cgi, can be used to obtain an optimal alignment of
peptidic or nucleotidic sequences and to calculate the percentage of sequence identity.

### Abbreviations used

- bp: base pair
- kb: kilo base
- BSA: bovine serum albumin
- DNA: deoxyribonucleic acid
- cDNA: complementary DNA
- DTT: dithiothreitol
- FID: Flame ionization detector
- FPP: farnesyl pyrophosphate
- GC: gaseous chromatograph
- IPTG: isopropyl-D-thiogalacto-pyranoside
- LB: lysogeny broth
- MS: mass spectrometer
- MVA: mevalonic acid
- PCR: polymerase chain reaction
- RMCE: recombinase-mediated cassette exchange
- 3'-/5'-RACE 3' and 5': rapid amplification of cDNA ends
- RNA: ribonucleic acid
- mRNA: messenger ribonucleic acid
- miRNA: micro RNA
- siRNA: small interfering RNA
- rRNA: ribosomal RNA
- tRNA: transfer RNA

### DEFINITIONS

The term "polypeptide" means an amino acid sequence of consecutively polymerized amino acid residues, for instance, at least 15 residues, at least 30 residues, at least 50 residues. In some embodiments provided herein, a polypeptide comprises an amino acid sequence that is an enzyme, or a fragment, or a variant thereof, as defined in the claims.

The term "isolated" polypeptide refers to an amino acid sequence that is removed from its natural environment by any method or combination of methods known in the art and includes recombinant, biochemical and synthetic methods.

The term "protein" refers to an amino acid sequence of any length wherein amino acids are linked by covalent peptide bonds, and includes oligopeptide, peptide, polypeptide and full length protein whether naturally occurring or synthetic.

The terms "drimenol synthase" or "drimenol synthase protein" refer to an enzyme that is capable of converting farnesyl diphosphate (FPP) to drimenol.

The terms "biological function," "function," "biological activity" or "activity" refer to the ability of the drimenol synthase to catalyze the formation of drimenol from FPP.

The terms "nucleic acid sequence," "nucleic acid," and "polynucleotide" are used interchangeably meaning a sequence of nucleotides. A nucleic acid sequence may be a single-stranded or double-stranded deoxyribonucleotide, or ribonucleotide of any length, and include coding and non- coding sequences of a gene, exons, introns, sense and anti-sense complimentary sequences, genomic DNA, cDNA, miRNA, siRNA, mRNA, rRNA, tRNA, recombinant nucleic acid sequences, isolated and purified naturally occurring DNA and/or RNA sequences, synthetic DNA and RNA sequences, fragments, primers and nucleic acid probes. The skilled artisan is aware that the nucleic acid sequences of RNA are identical to the DNA sequences with the difference of thymine (T) being replaced by uracil (U).

An "isolated nucleic acid" or "isolated nucleic acid sequence" is defined as a nucleic acid or nucleic acid sequence that is in an environment different from that in which the nucleic acid or nucleic acid sequence naturally occurs. The term "naturally-occurring" as used herein as applied to a nucleic acid refers to a nucleic acid that is found in a cell in nature. For example, a nucleic acid sequence that is present in an organism, for instance in the cells of an organism, that can be isolated from a source in nature and which has not been intentionally modified by a human in the laboratory is naturally occurring.

"Recombinant nucleic acid sequences" are nucleic acid sequences that result from the use of laboratory methods (molecular cloning) to bring together genetic material from more than one source, creating a nucleic acid sequence that does not occur naturally and would not be otherwise found in biological organisms.

"Recombinant DNA technology" refers to molecular biology procedures to prepare a recombinant nucleic acid sequence as described, for instance, in Laboratory Manuals edited by Weigel and Glazebrook, 2002 Cold Spring Harbor Lab Press; and Sambrook et al., 1989 Cold Spring Harbor, NY: Cold Spring Harbor Laboratory Press.

The term "gene" means a DNA sequence comprising a region, which is transcribed into a RNA molecule, e.g., an mRNA in a cell, operably linked to suitable regulatory regions, e.g., a promoter. A gene may thus comprise several operably linked sequences, such as a promoter, a 5' leader sequence comprising, e.g., sequences involved in translation initiation, a coding region of cDNA or genomic DNA, introns, exons, and/or a 3'non-translated sequence comprising, e.g., transcription termination sites.

A "chimeric gene" refers to any gene, which is not normally found in nature in a species, in particular, a gene in which one or more parts of the nucleic acid sequence are present that are not associated with each other in nature. For example the promoter is not associated in nature with part or all of the transcribed region or with another regulatory region. The term "chimeric gene" is understood to include expression constructs in which a promoter or transcription regulatory sequence is operably linked to one or more coding sequences or to an antisense, i.e., reverse complement of the sense strand, or inverted repeat sequence (sense and antisense, whereby the RNA transcript forms double stranded RNA upon transcription).

A "3' UTR" or "3' non-translated sequence" (also referred to as "3' untranslated region," or "3'end") refers to the nucleic acid sequence found downstream of the coding sequence of a gene, which comprises for example a transcription termination site and (in most, but not all eukaryotic imRNAs) a polyadenylation signal such as AAUAAA or variants thereof. After termination of transcription, the mRNA transcript may be cleaved downstream of the polyadenylation signal and a poly(A) tail may be added, which is involved in the transport of the mRNA to the site of translation, e.g., cytoplasm.

"Expression of a gene" involves transcription of the gene and translation of the mRNA into a protein. Overexpression refers to the production of the gene product as measured by levels of mRNA, polypeptide and/or enzyme activity in transgenic cells or organisms that exceeds levels of production in non-transformed cells or organisms of a similar genetic background.

"Expression vector" as used herein means a nucleic acid molecule engineered using molecular biology methods and recombinant DNA technology for delivery of foreign or exogenous DNA into a host cell. The expression vector typically includes sequences required for proper transcription of the nucleotide sequence. The coding region usually codes for a protein of interest but may also code for an RNA, e.g., an antisense RNA, siRNA and the like.

An "expression vector" as used herein includes any linear or circular recombinant vector including but not limited to viral vectors, bacteriophages and plasmids. The skilled person is capable of selecting a suitable vector according to the expression system. In one embodiment, the expression vector includes the nucleic acid of an embodiment herein operably linked to at least one regulatory sequence, which controls transcription, translation, initiation and termination, such as a transcriptional promoter, operator or enhancer, or an mRNA ribosomal binding site and, optionally, including at least one selection marker. Nucleotide sequences are "operably linked" when the regulatory sequence functionally relates to the nucleic acid of an embodiment herein. "Regulatory sequence" refers to a nucleic acid sequence that determines expression level of the nucleic acid sequences of an embodiment herein and is capable of regulating the rate of transcription of the nucleic acid sequence operably linked to the regulatory sequence. Regulatory sequences comprise promoters, enhancers, transcription factors, promoter elements and the like.

"Promoter" refers to a nucleic acid sequence that controls the expression of a coding sequence by providing a binding site for RNA polymerase and other factors required for proper transcription including without limitation transcription factor binding sites, repressor and activator protein binding sites. The meaning of the term promoter also includes the term "promoter regulatory sequence".
Promoter regulatory sequences may include upstream and downstream elements that may influences transcription, RNA processing or stability of the associated coding nucleic acid sequence. Promoters include naturally- derived and synthetic sequences. The coding nucleic acid sequences is usually located downstream of the promoter with respect to the direction of the transcription starting at the transcription initiation site.

The term "constitutive promoter" refers to an unregulated promoter that allows for continual transcription of the nucleic acid sequence it is operably linked to.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous. The nucleotide sequence associated with the promoter sequence may be of homologous or heterologous origin with respect to the plant to be transformed. The sequence also may be entirely or partially synthetic. Regardless of the origin, the nucleic acid sequence associated with the promoter sequence will be expressed or silenced in accordance with promoter properties to which it is linked after binding to the polypeptide of an embodiment herein. The associated nucleic acid may code for a protein that is desired to be expressed or suppressed throughout the organism at all times or, alternatively, at a specific time or in specific tissues, cells, or cell compartment. Such nucleotide sequences particularly encode proteins conferring desirable phenotypic traits to the host cells or organism altered or transformed therewith.
More particularly, the associated nucleotide sequence leads to the production of drimenol in the organism. Particularly, the nucleotide sequence encodes drimenol synthase.

"Target peptide" refers to an amino acid sequence which targets a protein, or polypeptide to intracellular organelles, i.e., mitochondria, or plastids, or to the extracellular space (secretion signal peptide). A nucleic acid sequence encoding a target peptide may be fused to the nucleic acid sequence encoding the amino terminal end, e.g., N-terminal end, of the protein or polypeptide, or may be used to replace a native targeting polypeptide.

The term "primer" refers to a short nucleic acid sequence that is hybridized to a template nucleic acid sequence and is used for polymerization of a nucleic acid sequence complementary to the template.

As used herein, the term "host cell" or "transformed cell" refers to a cell (or organism) altered to harbor at least one nucleic acid molecule, for instance, a recombinant gene encoding a desired protein or nucleic acid sequence which upon transcription yields a drimenol synthase protein useful to produce drimenol. The host cell is particularly a bacterial cell, a fungal cell or a plant cell. The host cell may contain a recombinant gene which has been integrated into the nuclear or organelle genomes of the host cell. Alternatively, the host may contain the recombinant gene extra-chromosomally. Homologous sequences include orthologous or paralogous sequences. Methods of identifying orthologs or paralogs including phylogenetic methods, sequence similarity and hybridization methods are known in the art and are described herein.

Paralogs result from gene duplication that gives rise to two or more genes with similar sequences and similar functions. Paralogs typically cluster together and are formed by duplications of genes within related plant species. Paralogs are found in groups of similar genes using pair-wise Blast analysis or during phylogenetic analysis of gene families using programs such as CLUSTAL. In paralogs, consensus sequences can be identified characteristic to sequences within related genes and having similar functions of the genes.

Orthologs, or orthologous sequences, are sequences similar to each other because they are found in species that descended from a common ancestor. For instance, plant species that have common ancestors are known to contain many enzymes that have similar sequences and functions. The skilled artisan can identify orthologous sequences and predict the functions of the orthologs, for example, by constructing a polygenic tree for a gene family of one species using CLUSTAL or BLAST programs. A method for identifying or confirming similar functions among homologous sequences is by comparing of the transcript profiles in plants overexpressing or lacking (in knockouts/knockdowns) related polypeptides. The skilled person will understand that genes having similar transcript profiles, with greater than 50% regulated transcripts in common, or with greater than 70% regulated transcripts in common, or greater than 90% regulated transcripts in common will have similar functions. Homologs, paralogs, orthologs and any other variants of the sequences disclosed herein are expected to function in a similar manner by making plants producing drimenol synthase proteins.

An embodiment provided herein provides amino acid sequences of drimenol synthase proteins.

The term "selectable marker" refers to any gene which upon expression may be used to select a cell or cells that include the selectable marker. Examples of selectable markers are described below. The skilled artisan will know that different antibiotic, fungicide, auxotrophic or herbicide selectable markers are applicable to different target species.

"Drimenol" for purposes of this application refers to (-)-drimenol (CAS: 468-68-8).

The term "organism" refers to any non-human multicellular or unicellular organisms such as a plant, or a microorganism. Particularly, a micro-organism is a bacterium, a yeast, an algae or a fungus. The term "plant" is used interchangeably to include plant cells including plant protoplasts, plant tissues, plant cell tissue cultures giving rise to regenerated plants, or parts of plants, or plant organs such as roots, stems, leaves, flowers, pollen, ovules, embryos, fruits and the like. Any plant can be used to carry out the methods of an embodiment herein.

The polypeptide to be contacted with farnesyl diphosphate (FPP), *in vitro* can be obtained by extraction from any organism expressing it, using standard protein or enzyme extraction technologies. If the host organism is an unicellular organism or cell releasing the polypeptide into the culture medium, the polypeptide may simply be collected from the culture medium, for example by centrifugation, optionally followed by washing steps and re-suspension in suitable buffer solutions. If the organism or cell accumulates the polypeptide within its cells, the polypeptide may be obtained by disruption or lysis of the cells and further extraction of the polypeptide from the cell lysate.

The polypeptide having a drimenol synthase activity, either in an isolated form or together with other proteins, for example in a crude protein extract obtained from cultured cells or microorganisms, may then be suspended in a buffer solution at optimal pH. If adequate, salts, DTT, inorganic cations and other kinds of enzymatic co-factors, may be added in order to optimize enzyme activity. The FPP is added to the polypeptide suspension, which is then incubated at optimal temperature, for example between 15 and 40°C, particularly between 25 and 35°C, more particularly at 30°C. After incubation, the drimenol produced may be isolated from the incubated solution by standard isolation procedures, such as solvent extraction and distillation, optionally after removal of polypeptides from the solution.

According to another particularly embodiment, the method of any of the above-described embodiments is carried out *in vivo.* In this case, step a) comprises cultivating a non-human host organism or cell capable of producing FPP and transformed to express at least one polypeptide comprising an amino acid sequence at least 70% identical to a sequence of SEQ ID NO: 2 and having a drimenol synthase activity, under conditions conducive to the production of drimenol.

According to a more particular embodiment, the method further comprises, prior to step a), transforming a non-human organism or cell capable of producing FPP with at least one nucleic acid encoding a polypeptide comprising an amino acid sequence at least 70% identical to a sequence of SEQ ID NO: 2 and having a drimenol synthase activity, so that said organism expresses said polypeptide.

These embodiments provided herein are particularly advantageous since it is possible to carry out the method *in vivo* without previously isolating the polypeptide. The reaction occurs directly within the organism or cell transformed to express said polypeptide.

According to a more particular embodiment at least one nucleic acid used in any of the above embodiments comprises a nucleotide sequence that has been obtained by modifying SEQ ID NO: 1 and SEQ ID NO: 3. Also disclosed herein are nucleic acids comprising a nucleotide sequence that has been obtained by modifying SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof. The at least one nucleic acid may be isolated from a plant of the Winteraceae family or the Canellaceae family, particularly from *Drimys Winteri* or *Drimys lanceolata.*

The organism or cell is meant to "express" a polypeptide, provided that the organism or cell is transformed to harbor a nucleic acid encoding said polypeptide, this nucleic acid is transcribed to mRNA and the polypeptide is found in the host organism or cell. The term "express" encompasses "heterologously express" and "over-express", the latter referring to levels of mRNA, polypeptide and/or enzyme activity over and above what is measured in a non-transformed organism or cell. A more detailed description of suitable methods to transform a non-human host organism or cell will be described later on in the part of the specification that is dedicated to such transformed non-human host organisms or cells.

A particular organism or cell is meant to be "capable of producing FPP" when it produces FPP naturally or when it does not produce FPP naturally but is transformed to produce FPP, either prior to the transformation with a nucleic acid as described herein or together with said nucleic acid. Organisms or cells transformed to produce a higher amount of FPP than the naturally occurring organism or cell are also encompassed by the "organisms or cells capable of producing FPP". Methods to transform organisms, for example microorganisms, so that they produce FPP are already known in the art.

To carry out an embodiment herein *in vivo,* the host organism or cell is cultivated under conditions conducive to the production of drimenol. Accordingly, if the host is a transgenic plant, optimal growth conditions are provided, such as optimal light, water and nutrient conditions, for example. If the host is a unicellular organism, conditions conducive to the production of drimenol may comprise addition of suitable cofactors to the culture medium of the host. In addition, a culture medium may be selected, so as to maximize drimenol synthesis. Optimal culture conditions are described in a more detailed manner in the following Examples.

Non-human host organisms suitable to carry out the method of an embodiment herein *in vivo* may be any non-human multicellular or unicellular organisms. In a particular embodiment, the non- human host organism used to carry out an embodiment herein *in vivo* is a plant, a prokaryote or a fungus. Any plant, prokaryote or fungus can be used. Particularly useful plants are those that naturally produce high amounts of terpenes. In a more particular embodiment the non-human host organism used to carry out the method of an embodiment herein *in vivo* is a microorganism. Any microorganism can be used but according to an even more particular embodiment said microorganism is a bacteria or yeast. Most particularly, said bacterium is *E. coli* and said yeast is *Saccharomyces cerevisiae.*

Some of these organisms do not produce FPP naturally. To be suitable to carry out the method of an embodiment herein, these organisms have to be transformed to produce said FPP. They can be so transformed either before the modification with the nucleic acid described according to any of the above embodiments or simultaneously, as explained above.

Isolated higher eukaryotic cells can also be used, instead of complete organisms, as hosts to carry out the method of an embodiment herein *in vivo.* Suitable eukaryotic cells may be any non-human cell, but are particularly plant or fungal cells.

In all embodiments, the polypeptide consists of an amino acid sequence having at least 70%, particularly at least 75%, particularly at least 80%, particularly at least 85%, particularly at least 90%, particularly at least 95%, particularly at least 96%, particularly at least 97%, particularly at least 98% and even more particularly at least 99% sequence identity to an amino acid sequence of SEQ ID NO: 2.

Also disclosed herein is a polypeptide having a drimenol synthase activity that comprises an amino acid sequence that is a variant of SEQ ID NO: 2, SEQ ID NO: 5, SEQ ID NO: 8, SEQ ID NO: 11 or SEQ ID NO: 14 obtained by genetic engineering, provided that said variant keeps its drimenol synthase activity, as defined above and has the required percentage of identity to SEQ ID NO: 2, SEQ ID NO: 5, SEQ ID NO: 8, SEQ ID NO: 11 or SEQ ID NO: 14. In other terms, said polypeptide particularly comprises an amino acid sequence encoded by a nucleotide sequence that has been obtained by modifying SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof. Also disclosed herein is a polypeptide having a drimenol synthase activity that consists of an amino acid sequence that is a variant of SEQ ID NO: 2, SEQ ID NO: 5, SEQ ID NO: 8, SEQ ID NO: 11 or SEQ ID NO: 14 obtained by genetic engineering, i.e. an amino acid sequence encoded by a nucleotide sequence that has been obtained by modifying SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof.

Also disclosed herein is a polypeptide having a drimenol synthase activity that is a variant of SEQ ID NO: 2, SEQ ID NO: 5, SEQ ID NO: 8, SEQ ID NO: 11 or SEQ ID NO: 14 that can be found naturally in other organisms, such as other plant species, provided that it keeps its drimenol synthase activity as defined above and has the required percentage of identity to of SEQ ID NO: 2, SEQ ID NO: 5, SEQ ID NO: 8, SEQ ID NO: 11 or SEQ ID NO: 14.

Examples of variant polypeptides are naturally occurring proteins that result from alternate mRNA splicing events or from proteolytic cleavage of the polypeptides described herein. Variations attributable to proteolysis include, for example, differences in the N- or C- termini upon expression in different types of host cells, due to proteolytic removal of one or more terminal amino acids from the polypeptides herein. Polypeptides encoded by a nucleic acid obtained by natural or artificial mutation of a nucleic acid of an embodiment herein, as described thereafter, are also disclosed herein.

Polypeptide variants resulting from a fusion of additional peptide sequences at the amino and carboxyl terminal ends can also be used in the methods disclosed. In particular such a fusion can enhance expression of the polypeptides, be useful in the purification of the protein or improve the enzymatic activity of the polypeptide in a desired environment or expression system. Such additional peptide sequences may be signal peptides, for example. Accordingly, disclosed herein are methods using variant polypeptides, such as those obtained by fusion with other oligo- or polypeptides and/or those which are linked to signal peptides. Polypeptides resulting from a fusion with another functional protein, such as another protein from the terpene biosynthesis pathway, can also be advantageously be used in the methods disclosed.

The at least one polypeptide having a drimenol synthase activity used in any of the above-described methods or encoded by the nucleic acid as disclosed may be isolated from a plant of the Winteraceae family or the Canellaceae family, particularly from *Drimys Winteri* or *Drimys lanceolata..*

An important tool to carry out the method of an embodiment herein is the polypeptide itself. A polypeptide having a drimenol synthase activity and comprising an amino acid sequence at least 70% identical to SEQ ID NO:2 is therefore provided herein.

The polypeptide may be capable of producing a mixture of sesquiterpenes wherein drimenol represents at least 20%, particularly at least 30% particularly at least 35%, particularly at least 90%, particularly at least 95%, more particularly at least 98% of the sesquiterpenes produced. In another aspect provided here, the drimenol is produced with greater than or equal to 95%, more particularly 98% selectivity.

According to the invention, the polypeptide comprises an amino acid sequence at least 70%, particularly at least 75%, particularly at least 80%, particularly at least 85%, particularly at least 90%, particularly at least 95%, particularly at least 96%, particularly at least 97%, particularly at least 98% and even more particularly at least 99% identical to a sequence of SEQ ID NO: 2.
According to a more particular embodiment, the polypeptide comprises an amino acid sequence of SEQ ID NO: 2.

According to another particular embodiment, the polypeptide consists of an amino acid sequence at least 70%, particularly at least 75%, particularly at least 80%, particularly at least 85%, particularly at least 90%, particularly at least 95%, particularly at least 96%, particularly at least 97%, particularly at least 98% and even more particularly at least 99% identical to a sequence of SEQ ID NO: 2.
According to a more particular embodiment, the polypeptide consists of an amino acid of SEQ ID NO: 2.

Also disclosed herein is a polypeptide that comprises an amino acid sequence that is a variant of SEQ ID NO: 2, either obtained by genetic engineering or found naturally in *Drimys* plants or in other plant species. In other terms, when the variant polypeptide is obtained by genetic engineering, said polypeptide comprises an amino acid sequence encoded by a nucleotide sequence that has been obtained by modifying SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof.
Also disclosed herein is a polypeptide having a drimenol synthase activity that consists of an amino acid sequence that is a variant of SEQ ID NO: 2, SEQ ID NO: 5, SEQ ID NO: 8, SEQ ID NO: 11 or SEQ ID NO: 14 obtained by genetic engineering, i.e. an amino acid sequence encoded by a nucleotide sequence that has been obtained by modifying SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 or the complement thereof.

The polypeptide may be isolated from a plant of the Winteraceae family or the Canellaceae family, particularly from *Drimys Winteri* or *Drimys lanceolata.*

As mentioned above, the nucleic acid encoding the polypeptide of an embodiment herein is a useful tool to modify non-human host organisms or cells intended to be used when the method is carried out *in vivo*.

A nucleic acid encoding a polypeptide having a drimenol synthase activity comprising an amino acid sequence having at least 70% sequence identity to an amino acid sequence of SEQ ID NO: 2 is therefore also provided herein.

The nucleic acid comprises a nucleotide sequence at least 50%, particularly at least 55%, particularly at least 60%, particularly at least 65%, particularly at least 70%, particularly at least 75%, particularly at least 80%, particularly at least 85%, particularly at least 90%, more particularly at least 95% particularly at least 96%, particularly at least 97%, particularly at least 98%, and even more particularly at least 99% identical to a sequence selected from the group consisting of SEQ ID NO: 1 or SEQ ID NO: 3 or the complement thereof. According to a more particular embodiment, the nucleic acid comprises the nucleotide sequence selected from the group consisting of SEQ ID NO: 1 or SEQ ID NO: 3.

The nucleic acid may consist of a nucleotide sequence at least 70%, particularly at least 75%, particularly at least 80%, particularly at least 85%, particularly at least 90%, particularly at least 95%, particularly at least 96%, particularly at least 97%, particularly at least 98% and even more particularly at least 99% or more identity to a sequence selected from the group consisting SEQ ID NO: 1 or SEQ ID NO: 3 or the complement thereof.
According to an even more particular embodiment, the nucleic acid consists of a sequence selected from the group consisting of SEQ ID NO: 1 or SEQ ID NO: 3 or the complement thereof.

The nucleic acid of an embodiment herein can be defined as including deoxyribonucleotide or ribonucleotide polymers in either single- or double-stranded form (DNA and/or RNA). The terms "nucleotide sequence" should also be understood as comprising a polynucleotide molecule or an oligonucleotide molecule in the form of a separate fragment or as a component of a larger nucleic acid.
Nucleic acids of an embodiment herein also encompass certain isolated nucleotide sequences including those that are substantially free from contaminating endogenous material.

The nucleic acid of an embodiment herein can be either present naturally in plants of the Drimys species or other species, or be obtained by modifying SEQ ID NO: 1 or SEQ ID NO: 3 or the complement thereof. Particularly said nucleic acid consists of a nucleotide sequence that has been obtained by modifying SEQ ID NO: 1 or SEQ ID NO: 3 or the complement thereof.

The nucleic acids comprising a sequence obtained by mutation of SEQ ID NO: 1 or SEQ ID NO: 3 or the complement thereof are encompassed by an embodiment herein, provided that the sequences they comprise share at least the defined percentage of identity with the corresponding sequences of SEQ ID NO: 1 or SEQ ID NO: 3 or the complement thereof and provided that they encode a polypeptide having a Drimenol synthase activity, as defined in any of the above embodiments. Mutations may be any kind of mutations of these nucleic acids, such as point mutations, deletion mutations, insertion mutations and/or frame shift mutations. A variant nucleic acid may be prepared in order to adapt its nucleotide sequence to a specific expression system. For example, bacterial expression systems are known to more efficiently express polypeptides if amino acids are encoded by particular codons.

Due to the degeneracy of the genetic code, more than one codon may encode the same amino acid sequence, multiple nucleic acid sequences can code for the same protein or polypeptide. Where appropriate, the nucleic acid sequences encoding the drimenol synthase may be optimized for increased expression in the host cell.
For example, nucleotides of an embodiment herein may be synthesized using codons particular by a host for improved expression.

Another important tool for transforming host organisms or cells suitable to carry out the method of an embodiment herein in vivo is an expression vector comprising a nucleic acid according to any embodiment of an embodiment herein. Such a vector is therefore also provided herein.

The expression vectors provided herein may be used in the methods for preparing a genetically transformed host organism and/or cell, in host organisms and/or cells harboring the nucleic acids of an embodiment herein and in the methods for making polypeptides having a drimenol synthase activity, as disclosed further below.

Recombinant non-human host organisms and cells transformed to harbor at least one nucleic acid of an embodiment herein so that it heterologously expresses or over-expresses at least one polypeptide of an embodiment herein are also very useful tools to carry out the method of an embodiment herein. Such non-human host organisms and cells are therefore also provided herein.

A nucleic acid according to any of the above-described embodiments can be used to transform the non-human host organisms and cells and the expressed polypeptide can be any of the above- described polypeptides.

Non-human host organisms of an embodiment herein may be any non-human multicellular or unicellular organisms. In a particular embodiment, the non-human host organism is a plant, a prokaryote or a fungus. Any plant, prokaryote or fungus is suitable to be transformed according to the methods provided herein. Particularly useful plants are those that naturally produce high amounts of terpenes.

In a more particular embodiment the non-human host organism is a microorganism. Any microorganism is suitable to be used herein, but according to an even more particular embodiment said microorganism is a bacteria or yeast. Most particularly, said bacterium is *E. coli* and said yeast is *Saccharomyces cerevisiae.*

Isolated higher eukaryotic cells can also be transformed, instead of complete organisms. As higher eukaryotic cells, we mean here any non-human eukaryotic cell except yeast cells. Particular higher eukaryotic cells are plant cells or fungal cells.

A variant may also differ from the polypeptide disclosed herein by attachment of modifying groups which are covalently or non-covalently linked to the polypeptide backbone. The variant also includes a polypeptide which differs from the polypeptide described herein by introduced N-linked or O-linked glycosylation sites, and/or an addition of cysteine residues. The skilled artisan will recognize how to modify an amino acid sequence and preserve biological activity. The functionality or activity of any drimenol synthase protein, variant or fragment, may be determined using various methods. For example, transient or stable overexpression in plant, bacterial or yeast cells can be used to test whether the protein has activity, i.e., produces drimenol from FPP. Drimenol synthase activity may be assessed in a microbial expression system, such as the assay described in Example 2 or 3 herein on the production of drimenol, indicating functionality. A variant or derivative of a drimenol synthase polypeptide disclosed herein retains an ability to produce drimenol from FPP. Amino acid sequence variants of the drimenol synthases disclosed herein may have additional desirable biological functions including, e.g., altered substrate utilization, reaction kinetics, product distribution or other alterations.

An embodiment herein provides polypeptides to be used in a method to produce drimenol by contacting an FPP with the polypeptides either *in vitro* or *in vivo.*

Disclosed herein is also an isolated, recombinant or synthetic polynucleotide encoding a polypeptide or variant polypeptide disclosed herein.

In an embodiment, an isolated, recombinant or synthetic nucleic acid sequence of SEQ ID NO: 1 or SEQ ID NO: 3 is provided encoding for a drimenol synthase having the amino acid sequence which is at least 70%, 75%, 80%, 85%, 90%, 92%, 95%, 96%, 97%, 98% or 99% identical to an amino acid of SEQ ID NO: 2 that catalyze production of drimenol in a cell from a FPP. Embodiments disclosed herein include, but are not limited to cDNA, genomic DNA and RNA sequences. Any nucleic acid sequence encoding the drimenol synthase is referred herein as a drimenol synthase encoding sequence.

According to a particular embodiment, the nucleic acid of SEQ ID NO: 1 or SEQ ID NO: 3, is the coding sequence of a drimenol synthase gene encoding the drimenol synthase obtained as described in the Examples.

A fragment of a polynucleotide of SEQ ID NO: 1, SEQ ID NO: 3, SEQ ID NO: 4, SEQ ID NO: 6, SEQ ID NO: 7, SEQ ID NO: 9, SEQ ID NO: 10 SEQ ID NO: 12, SEQ ID NO: 13 or SEQ ID NO: 15 refers to contiguous nucleotides that is particularly at least 15 bp, at least 30 bp, at least 40 bp, at least 50 bp and/or at least 60 bp in length of the polynucleotide disclosed herein. Particularly the fragment of a polynucleotide comprises at least 25, more particularly at least 50, more particularly at least 75, more particularly at least 100, more particularly at least 150, more particularly at least 200, more particularly at least 300, more particularly at least 400, more particularly at least 500, more particularly at least 600, more particularly at least 700, more particularly at least 800, more particularly at least 900, more particularly at least 1000 contiguous nucleotides of the polynucleotide. Without being limited, the fragment of the polynucleotides herein may be used as a PCR primer, and/or as a probe, or for anti-sense gene silencing or RNAi.

It is clear to the person skilled in the art that genes, including the polynucleotides of an embodiment herein, can be cloned on basis of the available nucleotide sequence information, such as found in the attached sequence listing, by methods known in the art. These include e.g. the design of DNA primers representing the flanking sequences of such gene of which one is generated in sense orientations and which initiates synthesis of the sense strand and the other is created in reverse complementary fashion and generates the antisense strand. Thermostable DNA polymerases such as those used in polymerase chain reaction are commonly used to carry out such experiments. Alternatively, DNA sequences representing genes can be chemically synthesized and subsequently introduced in DNA vector molecules that can be multiplied by e.g. compatible bacteria such as e.g. *E. coli.*

PCR primers and/or probes for detecting nucleic acid sequences encoding a drimenol synthase are disclosed. The skilled artisan will be aware of methods to synthesize degenerate or specific PCR primer pairs to amplify a nucleic acid sequence encoding the drimenol synthase, based on SEQ ID NO: 1 or SEQ ID NO: 3. ,. A detection kit for nucleic acid sequences encoding the drimenol synthase may include primers and/or probes specific for nucleic acid sequences encoding the drimenol synthase, and an associated protocol to use the primers and/or probes to detect nucleic acid sequences encoding the drimenol synthase in a sample. Such detection kits may be used to determine whether a plant has been modified, i.e., transformed with a sequence encoding the drimenol synthase.

Disclosed herein are nucleic acid sequences obtained by mutations of SEQ ID NO: 1 or SEQ ID NO: 3 such mutations can be routinely made. It is clear to the skilled artisan that mutations, deletions, insertions, and/or substitutions of one or more nucleotides can be introduced into the DNA sequence of SEQ ID NO: 1 or SEQ ID NO: 3. Generally, a mutation is a change in the DNA sequence of a gene that can alter the amino acid sequence of the polypeptide produced.

To test a function of variant DNA sequences disclosed herein, the sequence of interest is operably linked to a selectable or screenable marker gene and expression of the reporter gene is tested in transient expression assays with protoplasts or in stably transformed plants. The skilled artisan will recognize that DNA sequences capable of driving expression are built as modules. Accordingly, expression levels from shorter DNA fragments may be different than the one from the longest fragment and may be different from each other. Further disclosed herein are also functional equivalents of the nucleic acid sequence coding the drimenol synthase proteins, i.e., nucleotide sequences that hybridize under stringent conditions to the nucleic acid sequence of SEQ ID NO: 1 or SEQ ID NO: 3. The skilled artisan will be aware of methods to identify homologous sequences in other organisms and methods (identified in the Definition section herein) to determine the percentage of sequence identity between homologous sequences. Such newly identified DNA molecules then can be sequenced and the sequence can be compared with the nucleic acid sequence of SEQ ID NO: 1 or SEQ ID NO: 3 and tested for functional equivalence. Disclosed herein are DNA molecules having at least 70% particularly 75%, particularly 80%, particularly 85%, particularly 90%, particularly 95%, particularly 96% particularly 97% particularly 98%, or more particularly 99% or more sequence identity to the nucleotide sequence of SEQ ID NO: 1 or SEQ ID NO: 3.

Also disclosed is a nucleic acid sequence which is complementary to the nucleic acid sequence according to SEQ ID NO:1 or SEQ ID NO:3, such as inhibitory RNAs, or nucleic acid sequence which hybridizes under stringent conditions to at least part of the nucleotide sequence according to NO: 1 or SEQ ID NO: 3.

Disclosed is a method to alter gene expression in a host cell. For instance, the polynucleotide of an embodiment herein may be enhanced or overexpressed or induced in certain contexts (e.g. following insect bites or stings or upon exposure to a certain temperature) in a host cell or host organism.

Alteration of expression of a polynucleotide disclosed herein also results in "ectopic expression" which is a different expression pattern in an altered and in a control or wild-type organism. Alteration of expression occurs from interactions of polypeptide with exogenous or endogenous modulators, or as a result of chemical modification of the polypeptide. The term also refers to an altered expression pattern of the polynucleotide which is altered below the detection level or completely suppressed activity.

Disclosed are several drimenol synthase encoding nucleic acid sequences co-expressed in a single host, particularly under control of different promoters. Alternatively, several drimenol synthase protein encoding nucleic acid sequences can be present on a single transformation vector or be co-transformed at the same time using separate vectors and selecting transformants comprising both chimeric genes. Similarly, one or more drimenol synthase encoding genes may be expressed in a single plant together with other chimeric genes, for example encoding other proteins which enhance insect pest resistance, or others.

The nucleic acid sequences of an embodiment herein encoding drimenol synthase proteins can be inserted in expression vectors and/or be contained in chimeric genes inserted in expression vectors, to produce drimenol synthase proteins in a host cell or host organism. The vectors for inserting transgenes into the genome of host cells are well known in the art and include plasmids, viruses, cosmids and artificial chromosomes. Binary or co-integration vectors into which a chimeric gene is inserted are also used for transforming host cells.

Disclosed are recombinant expression vectors comprising a nucleic acid sequence of a drimenol synthase gene, or a chimeric gene comprising a nucleic acid sequence of a drimenol synthase gene, operably linked to associated nucleic acid sequences such as, for instance, promoter sequences. For example, a chimeric gene comprising a nucleic acid sequence of SEQ ID NO:1 or SEQ ID NO:3 may be operably linked to a promoter sequence suitable for expression in plant cells, bacterial cells or fungal cells, optionally linked to a 3' non-translated nucleic acid sequence.

Alternatively, the promoter sequence may already be present in a vector so that the nucleic acid sequence which is to be transcribed is inserted into the vector downstream of the promoter sequence. Vectors are typically engineered to have an origin of replication, a multiple cloning site, and a selectable marker.

### Examples

### Example 1

### Drimys lanceolata and Drimys winteri plant material and leaf transcriptome sequencing.

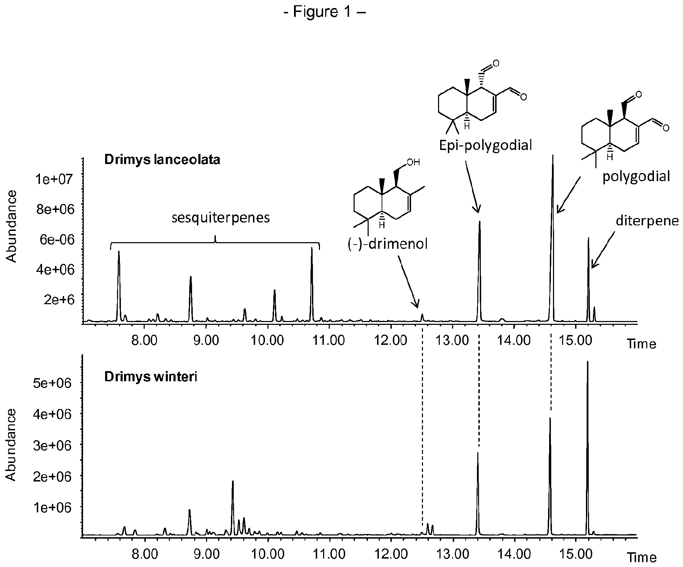

*Drimys winteri* and *Drimys lanceolata* plants were obtained from Bluebell Nursery (Leicestershire, UK). For analysis of the composition in terpene molecules, the leaves were collected and solvent extracted using MTBE (methyl tert-butyl ether). The extract was analyzed by GCMS using an Agilent 6890 Series GC system connected to an Agilent 5975 mass detector. The GC was equipped with 0.25 mm inner diameter by 30 m DB-1ms capillary column (Agilent). The carrier gas was He at a constant flow of 1 mL/min. The initial oven temperature was 50°C (1 min hold) followed by a gradient of 10°C/min to 300°C. The injection was made in a split/splitless injector set at 260°C and used in splitless mode.The identification of the products was based on the comparison of the mass spectra and retention indices with authentic standards and internal mass spectra databases. The leaves of the two plants contained significant quantities of drimane sesquiterpene compounds including (-)-drimenol, polygodial and epipolygodial (Figure 1).

Small leaves of *D. winteri* and *D. lanceolata* were thus taken for transcriptome analysis. Total RNA was extracted using the Concert™ Plant RNA Reagent (Invitrogen). This total RNA was processed using the Illumina Total RNA-Seq technique and the Illumina HiSeq 2000 sequencer. A total of 101 and 105 millions of paired-reads of 2x100 bp were generated for *D. winteri* and *D*. *lanceolata*, respectively. The reads were assembled using the Velvet de novo genomic assembler (http://www.ebi.ac.uk/∼zerbino/velvet/) and the Oases software (http://www.ebi.ac.uk/∼zerbino/oases/). For *D*. *winteri* 40'586 contigs with an average size of 1'080 bp were assembled and for *D. lanceolata* 28'255 contigs with an average size of 1'179 bp were obtained. The contigs were search using the tBlastn algorithm (Altschul et al, J. Mol. Biol. 215, 403-410, 1990) and using as query the amino acid sequences of known sesquiterpene synthases. This approach provided the sequences for 37 new putative sesquiterpene synthases. The enzymatic activity of these synthases were evaluated as described in the following examples for the synthases showing drimenol synthase activity.

### Example 2. Functional expression and characterization of DlTps589 from D. lanceolata.

The DNA sequences of one of the selected sesquiterpene synthases DlTps589 was codon-optimized, synthesized *in-vitro* and cloned in the pJ444-SR expression plasmid (DNA2.0, Menlo Park, CA, USA).

Heterologous expression of the DlTps589 synthases was performed in KRX *E. coli* cells (Promega). Single colonies of cells transformed with the pJ444SR-DlTps589 expression plasmid were used to inoculate 5 ml LB medium. After 5 to 6 hours incubation at 37°C, the cultures were transferred to a 20°C incubator and left 1 hour for equilibration. Expression of the protein was then induced by the addition of 1 mM IPTG and 0.2% L-rhamnose and the culture was incubated overnight at 20°C. The next day, the cells were collected by centrifugation, resuspended in 0.1 volume of 50 mM MOPSO pH 7, 10% glycerol and lyzed by sonication. The extracts were cleared by centrifugation (30 min at 20,000 g) and the supernatants containing the soluble proteins were used for further experiments.

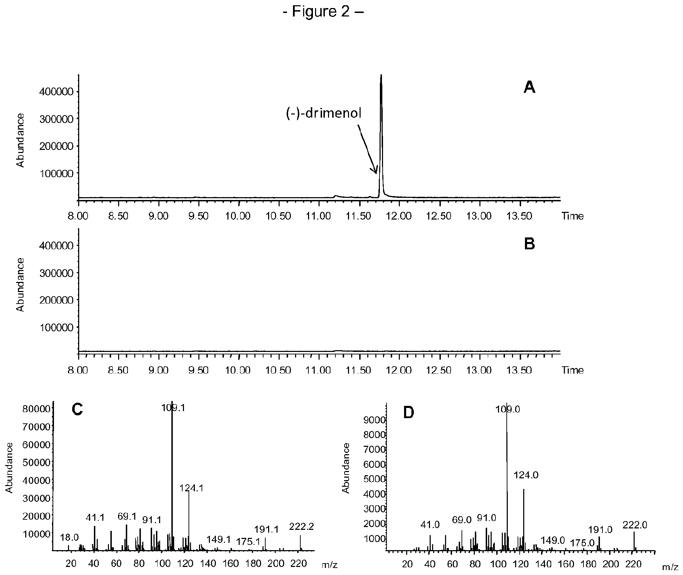

The crude *E coli* protein extracts containing the recombinant protein were used for the characterization of the enzymatic activities. The assays were performed in 2 mL of 50 mM MOPSO pH 7, 10% glycerol, 1 mM DTT, 15 mM MgCl₂ in the presence of 80 µM of farnesyl-diphosphate (FPP, Sigma) and 0.1 to 0.5 mg of crude protein. The tubes were incubated 12 to 24 hours at 30°C and extracted twice with one volume of pentane. After concentration under a nitrogen flux, the extracts were analyzed by GC and GC-MS and compared to extracts from assays with control proteins. The analysis of the products formed by the enzymes was made by GCMS as described in example 1. A negative control was performed in the same conditions using *E. coli* cells transformed with an empty pJ444 plasmid. In these conditions, the DlTps589 recombinant enzyme produced (-)-drimenol as major product with a selectivity over 98 % (Figure 2). The identity of (-)-drimenol was confirmed by matching of the mass spectrum and retention time of an authentic drimenol standard isolated from Sandalwood Oil West (*Amyris balsamifera*).

### Example 3. In vivo production of (-)-drimenol in E. coli cells using DlTps589

To evaluate the *in-vivo* production of (-)-drimenol in heterologous cells, *E. coli* cells were transformed with the pJ444SR-DlTps589 expression plasmid and the production of sesquiterpenes from the endogenous FPP pool was evaluated. To increase the productivity of the cells, an heterologous FPP synthase and an the enzymes from a complete heterologous mevalonate (MVA) pathway were also expressed in the same cells. The construction of the expression plasmid containing an FPP synthase gene and the gene for a complete MVA pathway was described in patent WO2013064411 or in Schalk et al (2013) J. Am. Chem. Soc. 134, 18900-18903. Briefly, an expression plasmid was prepared containing two operons composed of the genes encoding the enzymes for a complete mevalonate pathway. A first synthetic operon consisting of an *E. coli* acetoacetyl-CoA thiolase (atoB), a Staphylococcus aureus HMG-CoA synthase (mvaS), a Staphylococcus aureus HMG-CoA reductase (mvaA) and a Saccharomyces cerevisiae FPP synthase (ERG20) genes was synthetized *in-vitro* (DNA2.0, Menlo Park, CA, USA) and ligated into the *Nco*I*-Bam*HI digested pACYCDuet-1 vector (Invitrogen) yielding pACYC-29258. A second operon containing a mevalonate kinase (MvaK1), a phosphomevalonate kinase (MvaK2), a mevalonate diphosphate decarboxylase (MvaD), and an isopentenyl diphosphate isomerase (idi) was amplified from genomic DNA of Streptococcus pneumoniae (ATCC BAA-334) and ligated into the second multicloning site of pACYC-29258 providing the plasmid pACYC-29258-4506. This plasmid thus contains the genes encoding all enzymes of the biosynthetic pathway leading from acetyl-coenzyme A to FPP.

KRX *E. coli* cells (Promega) were co-transformed with the plasmid pACYC-29258-4506 and the plasmid pJ444SR-DlTps589. Transformed cells were selected on carbenicillin (50 µg/ml) and chloramphenicol (34 µg/ml) LB-agarose plates. Single colonies were used to inoculate 5 mL liquid LB medium supplemented with the same antibiotics. The culture was incubated overnight at 37°C. The next day 2 mL of TB medium supplemented with the same antibiotics were inoculated with 0.2 mL of the overnight culture. After 6 hours incubation at 37°C, the culture was cooled down to 28°C and 0.1 mM IPTG and 0.2% rhamnose were added to each tube. The cultures were incubated for 48 hours at 28°C. The cultures were then extracted twice with 2 volumes of MTBE, the organic phase were concentrated to 500 µL and analyzed by GC-MS as described above in Example 1.

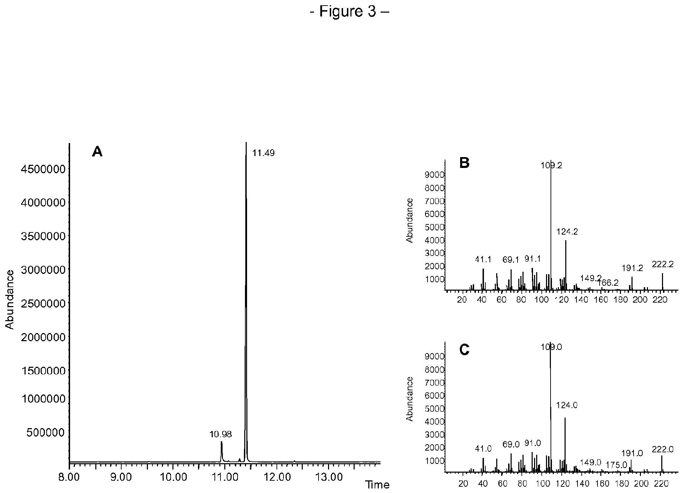

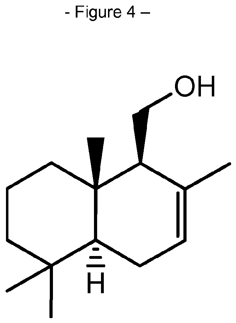

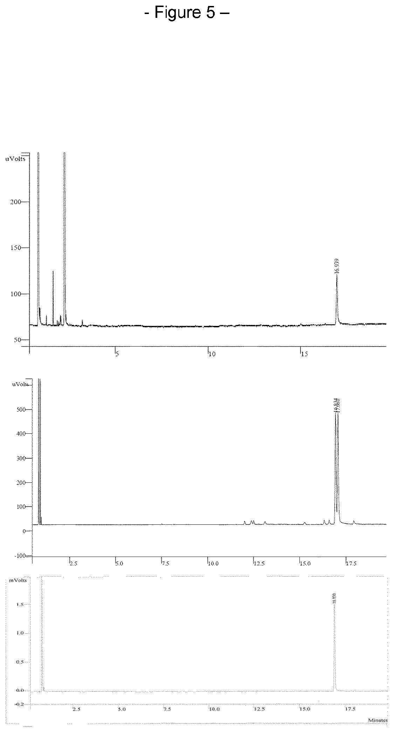

In this *in-vivo* conditions the DlTps589 recombinant enzyme produced (-)-drimenol as major product with the same apparent selectivity as in the *in vitro* assay described in example 2 (Figure 3). Using these engineered *E. Coli* cells larger (1 L) culture were used to purified the sesquiterpene produced by the enzyme in sufficient quantity to confirm the structure by NMR analysis and specific rotation measurement as being the structure of (-)-drimenol shown in figure 4. The enantiopurity was confirmed by chiral GC analysis on a Varian CP-3800 GC system equipped with a ChiraSil column (Agilent) and using oven gradient temperature of 3.0°C/min from 125 to 180°C (Figure 5).

### Example 4 (Reference Example). Functional expression and characterization of SCH51-3228-9 and SCH51-3228-11 from D. winteri.

SCH51-3228-9 and SCH51-3228-11 are two other DNA sequences putatively encoding for sesquiterpene synthases and isolated from the *Drymis winteri* transcriptome sequences. The deduced amino acid sequences share 92.6 and 95.1 % identity, respectively with the DlTps589 amino acid sequence. The two sequences were codon optimized, synthesized *in-vitro* (Invitrogen) and cloned between the *Nde*I and *Kpn*I restriction enzyme recognition sites of the pETDuet-1 (Novagen) expression plasmid (Invitrogen).

Heterologous expression of the SCH51-3228-9 and SCH51-3228-11 synthases was performed in BL21 (DE3) *E. coli* cells (Invitrogen). Single colonies of cells transformed with the pETDuet-SCH51- 3228-9 or the pEDTDuet-SCH51-3228-11 expression plasmids were used to produce the recombinant enzymes as described in example 2. The crude *E coli* protein extracts containing the recombinant proteins were used for the characterization of the enzymatic activities as described in example 2 except for the GCMS analysis conditions which were performed as follows. The GCMS analysis was made on an Agilent 6890 Series GC system connected to an Agilent 5975 mass detector. The GC was equipped with 0.25 mm inner diameter by 30 m DB-1ms capillary column (Agilent). The carrier gas was He at a constant flow of 1 mL/min. The initial oven temperature was 50°C (5 min hold) followed by a gradient of 5°C/min to 300°C. The injection was made in split mode at 250°C with a split ratio of 5:1.

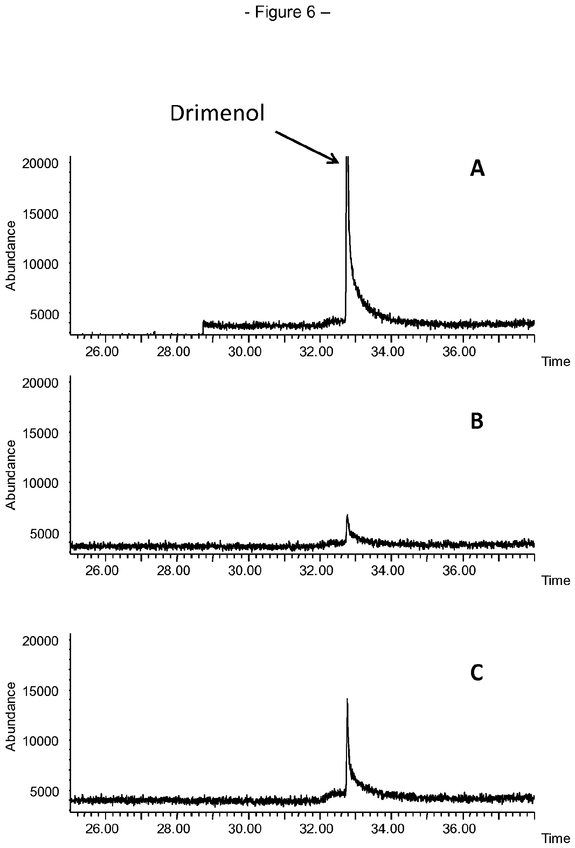

The two recombinant enzymes produced (-)-drimenol as major product with high selectivity. The identity of (-)-drimenol was confirmed by matching of the mass spectrum and retention time of an authentic drimenol standard isolated from Sandalwood Oil West (*Amyris balsamifera*) (Figure 6).

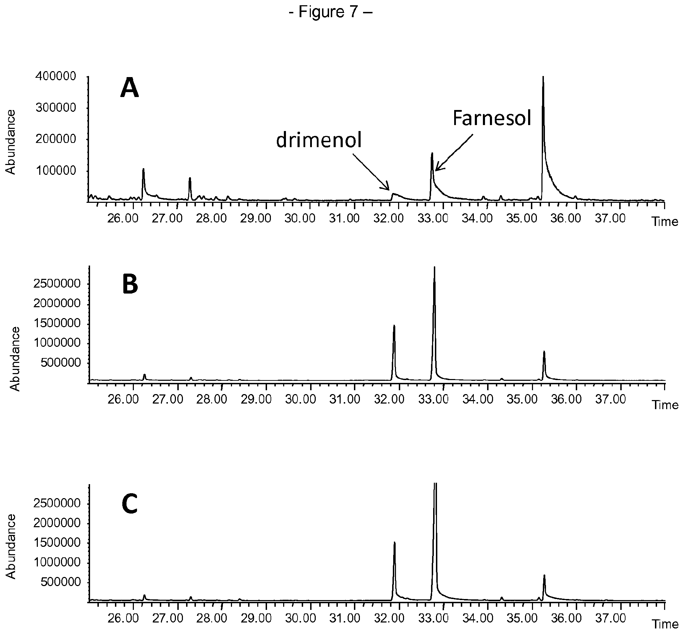

Using the whole *E. coli* cell system and method described in example 3 (except for the GCMS analysis conditions which were as described above) drimenol could also be produced in vivo in bacteria cultures using the SCH51-3228-9 and SCH51-3228-11 recombinant proteins (Figure 7).

### Example 5 (Reference Example). Functional expression and characterization of SCH51-998-28 from D. winteri and SCH52-13163-6 from D. lanceolata.

Similarly to example 4, the two cDNAs SCH51-998-28 and SCH52-13163-6 were optimized and cloned in the pETDuet expression plasmid.

The recombinant proteins were produced in in BL21 (DE3) *E. coli* cells (Invitrogen) and the *in vitro* assays using FPP as substrate were performed as described in example 2 and 4. These assays showed drimenol synthase activity for SCH51-998-28 and SCH52-13163-6 (figure 6). Using *E. Coli* cells overproducing FPP from a recombinant mevalonate pathway (example 2 and 4), drimenol could also be produced in vivo using the SCH51-998-28 and SCH52-13163-6 proteins (Figure 7).

### Example 6. Sequence comparison of the drimenol synthases from Drimys species.

The amino acid sequences of the drimenol synthases from *Drimys winteri* and *Drimys lanceolata* were aligned using the ClustalW Multiple alignment program (Thompson et al, 1994, Nucleic Acid Res.22(22), 4673-4680 and the sequence identities were calculated based on this alignment. Percent identity (%) between the different drimenol synthases from *Drimys* species:

| | DlTps589 | SCH51-3228-11 | SCH51-3228-9 | SCH51-998-28 | SCH52-13163-6 |
|---|---|---|---|---|---|
| DlTps589 | ID | 95.1 | 92.6 | 70.5 | 88 |
| SCH51_3228_11 | 95.1 | ID | 97.1 | 70.6 | 87.6 |
| SCH51_3228_9 | 92.6 | 97 | ID | 71 | 90.1 |
| SCH51_998_28 | 70.5 | 70.6 | 71 | ID | 72.5 |
| SCH52_13163_6 | 88 | 87.6 | 90.1 | 72.5 | ID |

### - Sequence listing -

SEQ ID NO.: 1
   SCH52-ctg589, Open reading frame of ClTps589 from *D. lanceolata,* wild type DNA sequence. 
ATGGATCTTATTAATCCCTCCCCAGCGGCTTCCACCCTCCCTCTCCCAGTTGATGGAGATTC
AGAAGTTGTTAGGCGATCTGCCGGGTTTCATCCGACTATCTGGGGCGATCACTTCCTCTCCT
ACAAGCCCGATCCAAAGAAAATAGATGCATGGAATAAAGGGTTGAAGAGCTGAAGGAAG
AAGTGAAGAAGATATTAAGCAATGCAAAAGGACGGTGGAAGAGCTGAATTTGATTGATG
ATCTCGTACACCTTGGGATTAGTTATCATTTTGAGAAGGAGATTGATGATGCTCTACAACAC
ATCTTTGATACCCATCTTGATGATTTTCCTAAGGATGATCTATATGTCGCCGCTCTCCGATTT
GGCGTCTTAAGGAAACAGGGGCACCGTGTTTCTCCAGATGTATTCAAAAAATTCAAAGATG
AGCAGGGGAATTTCAAGGCAGAGTTGAGCACCGATGCGAAAGGTTTGCTATGTTTAAATGA
TGTGGCTTATCTCAGCACAAGAGGGGAAGATATCTTGGATGAAGCCATTCCTTTCACTGAG
GAGCACCTTAGGTCTTGTATTAGCCATGTAGATTCTCATATGGCAGCAAAAATTGAACATTC
TCTCGAGCTTCCCCTTCATCATCGCATACCAAGGCTAGAGAACAGGCACTACATCTCAGTCT
ATGAAGGAGACAAGGAAAGGAACGAAGTTGTCCTTGAGCTTGCCAATTTAGATTTCAATCT
GATTCAAATCTTGCACCAAAGAGAGCTGAGAGACATCACAATGTGGTGGAAGGAGATTGA
CCTTGCAGCAAAGCTGCCTTTTATTAGGGATAGGTTGGTGGAGTGCTACTACTGGATCATG
GGGTCTATTTTGAACCAATATACTCGAGGGCTAGGGTTTTTCCACCAAAATGACAATGTT
GGTCTCAGTTGTGGACGACATATATGATGTGTATGCTACCGAGGATGAGCTTCAACTATTC
ACTGATGCCATCTATAGGTGGGATGCTGATGACATTGATCAGCTGCCTCAGTACTTGAAAG
ATGCTTTTATGGTACTCTACAACACTGTGAAGACTCTAGAAGAACTTGAACCAGAAGG
AAACTCTTATCGTGGATTCTATGTAAAAGATGCAATGAAGGTTTTGGCAAGGGATTACTTT
GTGGAGCACAAATGGTATAACAGAAAAATTGTGCCATCCGTAGAGGAATACTTGAAAATTT
CTTGCATCAGTGTGGCCGTTCATATGGCTACAGTTCACTGTATTGCTGGGATGTATGAAATT
GCAACCAAAGAGGCATTCGAATGGTTGATGACTGAGCCCAAACTTGTTATTGATGCATCTC
TGATTGGTCGTCTCCTTGATGACATGCAGTCCACCTCGTTTGAGCAACAGAGAGGCCACGT
GTCATCAGCAGTACAGTGTTACATGGCTGAATATGGTGTAACAGCGGAAGAAGCATGTGAA
AAGCTCCGAGATATGGCTGCAATTGCTTGGAAAGATGTGAACGAGGCATGCCTTAGGCCCA
CGGTTTTCCCTATGCCTATCCTTTTGCCTTCTATCAACTTGGCACGTGTGGCAGAAGTCATCT
 
ACCTACGTGGAGATGGATACACGCACGCTGGGGGTGAGACCAAGAAACACATCACGGCCA
TGCTTGTTAAGCCAATTGAAGTCTGA
SEQ ID NO.: 2
   ClTps589 from *D. lanceolata,* amino acid sequence. 
MDLINPSPAASTLPLPVDGDSEVVRRSAGFHPTIWGDHFLSYKPDPKKIDAWNKRVEELKEEVK
KILSNAKGTVEELNLIDDLVHLGISYHFEKEIDDALQHIFDTHLDDFPKDDLYVAALRFGVLRKQ
GHRVSPDVFKKFKDEQGNFKAELSTDAKGLLCLNDVAYLSTRGEDILDEAIPFTEEHLRSCISHV
DSHMAAKIEHSLELPLHHRIPRLENRHYISVYEGDKERNEVVLELANLDFNLIQILHQRELRDIT
MWWKEIDLAAKLPFIRDRLVECYYWIMGVYFEPIYSRARVFSTKMTMLVSVVDDIYDVYATE
DELQLFTDAIYRWDADDIDQLPQYLKDAFMVLYNTVKTLEEELEPEGNSYRGFYVKDAMKVL
ARDYFVEHKWYNRKIVPSVEEYLKISCISVAVHMATVHCIAGMYEIATKEAFEWLMTEPKLVID
ASLIGRLLDDMQSTSFEQQRGHVSSAVQCYMAEYGVTAEEACEKLRDMAAIAWKDVNEACLR
PTVFPMPILLPSINLARVAEVIYLRGDGYTHAGGETKKHITAMLVKPIEV
SEQ ID NO.: 3
   DlTps589_opt, Codon optimized DNA sequence of DlTps589 from *D. lanceolata.* 
ATGGACCTGATTAACCCGAGCCCTGCTGCATCCACCCTGCCACTGCCAGTCGATGGTGATA
GCGAAGTTGTGCGCCGTAGCGCGGTTTCCATCCGACCATCTGGGGTGACCACTTTCTGTCT
TATAAGCCGGACCCGAAAAAGATTGATGCGTGGAACAAGCGTGTTGAGGAACTGAAAGAA
GAGGTCAAAAAGATTTTGAGCAATGCGAAAGGCACGGTTGAGGAACTGAATTTGATTGAC
GACCTGGTACACCTGGGTATTAGCTATCACTTTGAGAAAGAAATCGACGACGCGCTGCAGC
ATATCTTCGATACGCACCTGGATGATTTCCCGAAAGATGACCTCTACGTGGCTGCGCTGCGT
TTTGGCGTCCTGCGTAAGCAAGGCCATCGTGTCAGCCCGGACGTCTTTAAGAAATTCAAAG
ACGAGCAAGGCAACTTCAAAGCGGAGCTGTCAACCGATGCAAAGGGCCTGTTGTGCCTGA
ACGATGTGGCGTACCTGAGCACCCGTGGTGAGGATATCCTGGACGAAGCGATCCCGTTCAC
GGAAGAACATTTGCGCTCGTGCATTAGCCACGTTGATAGCCACATGGCAGCGAAGATTGAG
CACTCTCTGGAGCTGCCGCTGCACCATCGCATTCCGCGTTTAGAGAATCGCCATTACATCTC
CGTGTACGAGGGTGACAAAGAGCGTAATGAAGTCGTTCTGGAGTTGGCTAACTTGGACTTT
AATCTTATCCAGATCCTGCACCAGCGCGAGCTGCGCGACATCACGATGTGGTGGAAAGAA
TTGATCTGGCCGCAAAGCTGCCGTTTATTCGTGACCGTCTGGTGGAGTGTTACTATTGGATT
 
ATGGGCGTGTACTTCGAGCCGATCTACAGCCGTGCGCGTGTTTAGCACCAAGATGACCA
TGCTGGTTAGCGTGGTGGATGACATCTATGATGTCTACGCTACGGAAGATGAGTTGCAGCT
GTTTACCGACGCCATTTACAGATGGGACGCCGATGACATTGATCAACTGCCGCAATATCTG
AAAGACGCCTTTATGGTTCTGTACAACACCGTCAAAACCCTGGAAGAAGAACTGGAGCCGG
AAGGTAACTCTTATCGTGGTTTCTACGTTAAAGATGCGATGAAAGTTCTGGCGCGTGACTAT
TTCGTTGAGCATAAGTGGTACAATCGTAAGATCGTCCCGTCCGTTGAAGAGTACTTGAAGA
TTAGCTGTATCAGCGTCGCAGTCCACATGGCGACCGTGCACTGTATCGCCGGCATGTATGA
GATCGCCACGAAAGAAGCATTCGAGTGGCTGATGACCGAGCCGAAACTGGTGATTGACGC
AAGCCTGATTGGTCGCCTGCTGGACGATATGCAGAGCACGAGCTTTGAGCAGCAGCGCGGT
CATGTTAGCTCCGCAGTTCAATGCTACATGGCTGAGTACGGTGTGACTGCCGAAGAAGCAT
GCGAGAAGCTGCGTGATATGGCGGCCATTGCGTGGAAAGATGTGAATGAAGCATGCCTGC
GCCCGACCGTTTTCCCGATGCCGATTTTACTGCCTAGCATCAACCTGGCACGTGTGGCGGA
AGTTATCTATCTGCGTGGCGACGGTTATACGCACGCGGGTGGTGAGACTAAGAAGCACATC
ACCGCGATGCTGGTCAAGCCGATCGAAGTGTAA
SEQ ID NO.: 4
   SCH51-3228-9 from *D. winteri*, Open reading frame , wild type DNA sequence. 
ATGGCTTCCACCCTCCCTCTCCCAGCTTATGGAGATTCAGAAGTTGTTAGGCGATCTGCCGG
GTTTCATCCGACGATCTGGGCGATCACTTCCTCTCCTACAAGCCTGATCCAACGAAAATA
GATGAATGGAATAAAAGGGTTGAAGAGCTGAAGGAAGAAGTGAAGAAGATATTAAGCAAT
GCAAAAGGACAGTGGAAGAGCTGAATTTGCTTGATGATCTCGTACACCTTGGGATTAGTT
ATCATTTTGAGAAGGAGATTGATGATGCTTTACAACAAATCTTTGATACCCATCTTGATGTT
TTTCCTAAGGATGATCTATATGCCACCGCTCTCCGATTTGGCGTCTTAAGGAAACAGGGGC
ACCGTGTTTCTCCAGATGTATTCAAAAAATTCAAAGATGAGCAGGGAATTTCAAGGCAGA
GTTGAGCACCGATGCGAAGGGTTTGCTATGTTTATATGATGTGGCTTATCTCAGCACAAGA
GGGGAAGATATCTTGGATGAAGCCATTCCTTTCACTAAGGAGCACCTTAGGTCTTGTATTA
GCCATGTCGATTCTCATATGGCAGCAAAAATTGAGCATTCTCTAGAGCTTCCCCTTCATCAT
CGCATACCAAGGCTAGAGAACAGGCACTACATCTCAGTCTATGAAGGAGACAAGGAAAGG
AATGAAGTTGTCCTTGAGCTTGCCAAATTAGATTTCAATCTGATTCAAATCTTGCACCAAAG
AGAGCTGAGGGACATCACAACGTGGTGGAAGGAGATTGACCTTGCAGCAAAGCTACCTTTT
 
ATTAGGGATAGGTTGGTGGAGTGCTACTATTGGATCATGGGAGTCTATTTTGAACCAATAT
ACTCAAGGGCTAGAGTTTTTCGACCAAAATGACAATCTTGGTCTCAGTTGTGGACGACAT
ATATGATGTATATGCTACAGAGGATGAGCTCCAACTTTTCACTGATGCAATCTATAGGTGG
GATGCTGAGGACATTGAGCAGCTTCCACAGTACTTGAAAGATGCTTTTCTTGTACTCTATAA
CACTGTGAAGGACCTAGAAGAGGAATTGGAACCAGAAGGAAACTCTTATCGTGGATACTA
TGTAAAAGATGCGATGAAGGTTTTGGCAAGGGATTACTTTGTGGAGCACAAATGGTATAAC
AGAAAAATTGTGCCATCAGTAGAGGACTACCTGCGAATTTCTTGCATTAGTGTTGCCGTTCA
TATGGCCACAGTTCATTGTATTGCTGGGATGTATGAAATTGCAACCAAAGAGGCATTCGAA
TGGTTGAAGACGGAACCTAAACTTGTTATAGATGCATCACTGATTGGGCGTCTCCTCGATG
ACATGCAGTCCACCTCGTTTGAGCAACAGAGAGGTCATGTGTCATCAGCGGTACAGTGTTA
CATGATCCAATATGGGGTATCACACGAAGAAGCGTGTGAGAAGTTGCGAGAAATGGCTGC
AATTGCGTGGAAAGATGTAAACCAAGCATGCCTTAGGCCCACTGTTTTCCCTATGCCTATTC
TTCTGCCCTCCATCAACCTTGCACGTGTGGCAGAAGTGATTTACCTACGCGGAGATGGATAT
ACACATGCGGGTGGTGAGACCAAAAAACATATCACGGCCATGCTTGTTGATCCAATCAAAG
TCTGA
SEQ ID NO.: 5
   SCH51-3228-9 from *D. winteri*, amino acid sequence. 
MASTLPLPAYGDSEVVRRSAGFHPTIWGDHFLSYKPDPTKIDEWNKRVEELKEEVKKILSNAKG
TVEELNLLDDLVHLGISYHFEKEIDDALQQIFDTHLDVFPKDDLYATALRFGVLRKQGHRVSPD
VFKKFKDEQGNFKAELSTDAKGLLCLYDVAYLSTRGEDILDEAIPFTKEHLRSCISHVDSHMAA
KIEHSLELPLHHRIPRLENRHYISVYEGDKERNEVVLELAKLDFNLIQILHQRELRDITTWWKEID
LAAKLPFIRDRLVECYYWIMGVYFEPIYSRARVFSTKMTILVSVVDDIYDVYATEDELQLFTDAI
YRWDAEDIEQLPQYLKDAFLVLYNTVKDLEEELEPEGNSYRGYYVKDAMKVLARDYFVEHK
WYNRKIVPSVEDYLRISCISVAVHMATVHCCAGMDEIATKEAFEWLKTEPKLVIDASLIGRLLD
DMQSTSFEQQRGHVSSAVQCYMIQYGVSHEEACEKLREMAAIAWKDVNQACLRPTVFPMPIL
LPSINLARVAEVIYLRGDGYTHAGGETKKHITAMLVDPIKV
SEQ ID NO.: 6
   SCH51-3228-9_opt, Codon optimized DNA sequence of SCH51-3228-9. 
ATGGCAAGCACCCTGCCGCTGCCTGCCTATGGTGATAGCGAAGTTGTTCGTCGTAGCGCAG
GTTTTCATCCGACCATTTGGGGTGATCATTTTCTGAGCTATAAACCGGATCCGACCAAAATT
GATGAATGGAATAAACGTGTCGAAGAACTGAAAGAAGTGAAAAAAATCCTGAGCAAT
GCCAAAGGCACCGTTGAGGAACTGAATCTGCTGGATGATCTGGTTCATCTGGGTATCAGCT
ATCACTTTGAGAAAGAAATCGATGATGCACTGCAGCAGATTTTTGATACCCATCTGGATGT
TTTCCCGAAAGATGATCTGTATGCAACCGCACTGCGTTTTGGTGTTCTGCGTAAACAGGGTC
ATCGTGTTAGTCCGGATGTGTTCAAAAAATTCAAAGATGAACAGGGCAACTTCAAAGCAGA
ACTGAGCACCGATGCAAAAGGTCTGCTGTGTCTGTATGATGTTGCATATCTGAGCACCCGT
GGTGAAGATATTCTGGATGAAGCAATTCCGTTTACCAAAGAACATCTGCGTAGCTGTATTA
GCCATGTTGATAGCCACATGGCAGCGAAAATTGAACATAGCCTGGAACTGCCTCTGCATCA
CCGTATTCCGCGTCTGGAAAATCGTCACTATATTAGCGTTTATGAGGGCGATAAAGAACGC
AATGAAGTTGTGCTGGAACTGGCAAAACTGGATTTTAACCTGATTCAGATTCTGCATCAGC
GTGAACTGCGTGATATTACCACCTGGTGGAAAGAAATTGATCTGGCAGCAAAACTGCCGTT
TATTCGTGATCGTCTGGTTGAATGCTATTATTGGATTATGGGCGTGTATTTCGAACCGATTT
ATAGCCGTGCACGTGTTTTTAGCACCAAAATGACCATTCTGGTTAGCGTGGTGGATGATATC
TATGATGTTTATGCCACCGAAGATGAACTGCAGCTGTTTACCGATGCCATTTATCGTTGGGA
TGCAGAAGATATTGAACAGCTGCCGCAGTATCTGAAAGATGCATTTCTGGTTCTGTACAAC
ACCGTGAAAGATCTGGAAGAAGAACTGGAACCGGAAGGTAATAGCTATCGTGGTTATTAT
GTTAAAGATGCCATGAAAGTTCTGGCACGCGATTATTTTGTTGAGCACAAATGGTATAACC
GCAAAATTGTTCCGAGCGTGGAAGATTATCTGCGTATTAGCTGCATTAGCGTTGCAGTTCAC
ATGGCAACCGTTCATTGTTGTGCAGGTATGGATGAAATTGCAACCAAAGAAGCATTTGAGT
GGCTGAAAACCGAACCGAAACTGGTTATTGATGCAAGCCTGATTGGTCGTCTGCTGGACGA
TATGCAGAGCACCAGCTTTGAACAGCAGCGTGGTCATGTTAGCAGCGCAGTTCAGTGTTAT
ATGATTCAGTATGGTGTTAGCCATGAAGAAGCATGCGAAAAACTGCGCGAAATGGCAGCA
ATTGCATGGAAAGATGTTAATCAGGCATGTCTGCGTCCGACCGTTTTTCCGATGCCGATTCT
GCTGCCGAGCATTAATCTGGCACGTGTTGCCGAAGTTATCTATCTGCGTGGTGATGGTTATA
CCCATGCCGGTGGTGAAACCAAAAAACATATTACCGCAATGCTGGTCGATCCGATTAAAGT
TTAA
SEQ ID NO.: 7
   SCH51-3228-11 from *D. winteri*, Open reading frame, wild type DNA sequence. 
ATGGCTTCCACCCTCCCTCTCCCAGCTTATGGAGATTCAGAAGTTGTTAGGCGATCTGCCGG
GTTTCATCCGACGATCTGGGGCGATCACTTCCTCTCCTACAAGCCTGATCCAACGAAAATA
GATGAATGGAATAAAAGGGTTGAAGAGCTGAAGGAAGAAGTGAAGAGATATTAAGCAAT
GCAAAAGGGACAGTGGAAGAGCTGAATTTGCTTGATGATCTCGTACACCTTGGGATTAGTT
ATCATTTTGAGAAGGAGATTGATGATGCTTTACAACAAATCTTTGATACCCATCTTGATGTT
TTTCCTAAGGATGATCTATATGCCACCGCTCTCCGATTTGGCGTCTTAAGGAAACAGGGGC
ACCGTGTTTCTCCAGATGTATTCAAAAAATTCAAAGATGAGCAGGGGAATTTCAAGGCAGA
GTTGAGCACCGATGCGAAGGGTTTGCTATGTTTATATGATGTGGCTTATCTCAGCACAAGA
GGGGAAGATATCTTGGATGAAGCCATTCCTTTCACTAAGGAGCACCTTAGGTCTTGTATTA
GCCATGTCGATTCTCATATGGCAGCAAAAATTGAGCATTCTCTAGAGCTTCCCCTTCATCAT
CGCATACCAAGGCTAGAGAACAGGCACTACATCTCAGTCTATGAAGGAGACAAGGAAAGG
AATGAAGTTGTCCTTGAGCTTGCCAAATTAGATTTCAATCTGATTCAAATCTTGCACCAAAG
AGAGCTGAGGGACATCACAATGTGGTGGAAGGAGATTGACCTTGCAGCAAAGCTACCTTTT
ATTAGAGATAGGTTGGTGGAGTGCTACTACTGGATCATGGGGGTCTATTTTGAACCAATAT
ACTCCAGGGCTAGGGTTTTTTCCACTAAAATGACAATCTTGGTCTCAGTTGTGGACGACATA
TATGATGTCTATGCTACGGAGGATGAGCTTCAACTATTCACTGATGCAATCTATAGGTGGG
ATGCTGATGACATTGATCAGCTGCCTCAGTACTTGAAAGATGCTTTTATGGTACTCTATAAC
ACTGTGAAGACTCTAGAAGAAGAACTTGAACCAGAAGGAAACTCTTATCGTGGATACTACG
TAAAAGATGCAATGAAGGTTTTGGCAAGAGATTACTTTGTGGAACACAAATGGTATAACAG
ACAAATTGTGCCATCCGTAGAGGAATACTTGAAAATTTCTTGCATTAGTGTGGCTGTTCATA
TGGCTACAGTTCATTGTATTGCTGGGATGTATGAAATTGCTACCAAAGAGGCATTCGAATG
GTTGAAGACTGAACCCAAACTTGTTATCGATGCATCTCTGATCGGTCGTCTTCTTGATGACA
TGCAGTCTACCTCGTTTGAGCAACAAAGAGGGCACGTGTCATCAGCAGTACAGTGTTACAT
GGCCCAATATGGAGTAACAGCAGAAGAAGCATGTGAAAAGCTACGAGAAATGGCTGCAAT
TGCTTGGAAAGATGTGAATGAAGCATGCCTTAGGCCCACGGTATTCCCTATGCCTATCCTCT
TGCCTTCTATCAACTTGGCACGTGTGGCAGAAGTGATCTACCTACGTGGAGATGGATACAC
GCACGCTGGGGGTGAGACCAAAAAACACATCACGGCCATGCTTGTTAAGCCAATTGAAGTC
TGA
SEQ ID NO.: 8
   SCH51-3228-11 from *D. winteri*, amino acid sequence. 
MASTLPLPAYGDSEVVRRSAGFHPTIWGDHFLSYKPDPTKIDEWNKRVEELKEEVKKILSNAKG
TVEELNLLDDLVHLGISYHFEKEIDDALQQIFDTHLDVFPKDDLYATALRFGVLRKQGHRVSPD
VFKKFKDEQGNFKAELSTDAKGLLCLYDVAYLSTRGEDILDEAIPFTKEHLRSCISHVDSHMAA
KIEHSLELPLHHRIPRLENRHYISVYEGDKERNEVVLELAKLDFNLIQILHQRELRDITMWWKEI
DLAAKLPFIRDRLVECYYWIMGVYFEPIYSRARVFSTKMTILVSVVDDIYDVYATEDELQLFTD
AIYRWDADDIDQLPQYLKDAFMVLYNTVKTLEEELEPEGNSYRGYYVKDAMKVLARDYFVEH
KWYNRQIVPSVEEYLKISCISVAVHMATVHCIAGMYEIATKEAFEWLKTEPKLVIDASLIGRLLD
DMQSTSFEQQRGHVSSAVQCYMAQYGVTAEEACEKLREMAAIAWKDVNEACLRPTVFPMPIL
LPSINLARVAEVIYLRGDGYTHAGGETKKHITAMLVKPIEV
SEQ ID NO.: 9
   SCH51-3228-11_opt from *D. winteri*, Codon optimized DNA sequence of SCH51-3228-11. 
ATGGCATCTACTCTTCCACTGCCGGCTTATGGTGATTCTGAGGTTGTTCGTCGTTCCGCGGG
TTTTCACCCTACCATCTGGGGCGATCACTTTCTGTCCTATAAGCCAGACCCGACCAAGATTG
ACGAGTGGAATAAGCGTGTCGAGGAACTGAAAGAAGTGAAAAAGATCCTGTCCAACG
CAAAAGGTACTGTCGAGGAGCTGAATCTGCTGGATGACCTGGTGCATCTGGGCATCAGCTA
TCACTTCGAAAAGGAAATTGACGACGCTTTGCAGCAAATTTTTGATACGCACCTGGACGTC
TTTCCGAAAGATGACCTGTATGCGACCGCGCTGCGCTTTGGTGTGCTGCGTAAACAGGGTC
ATCGCGTGTCTCCTGATGTGTTCAAGAAATTTAAAGATGAACAGGGCAATTTCAAGGCCGA
GTTGAGCACGGACGCCAAAGGTTTGCTCTGCCTGTACGACGTTGCATATCTGAGCACCCGT
GGTGAAGATATCCTGGACGAAGCGATTCCGTTCACCAAGGAACATCTGCGCTCGTGCATTT
CCCATGTAGATAGCCACATGGCGGCCAAGATCGAGCACAGCCTGGAGCTGCCTTTGCACCA
TCGTATTCCGCGCCTGGAGAATCGCCATTACATTAGCGTCTATGAGGGTGACAAAGAGCGC
AACGAAGTCGTGTTAGAGCTGGCGAAGCTGGACTTCAACCTGATTCAAATTCTGCATCAAC
GCGAGCTGCGCGACATTACCATGTGGTGGAAAGAGATTGATCTGGCAGCGAAGCTGCCGTT
 
CATCCGCGATCGTCTGGTTGAGTGCTACTACTGGATCATGGGCGTCTACTTCGAGCCGATCT
ACAGCCGCGCTCGTGTGTTTTCGACGAAGATGACCATCCTGGTTAGCGTTGTTGATGACATT
TATGACGTTTACGCGACCGAAGATGAACTGCAGCTGTTTACGGACGCAATCTACCGTTGGG
ACGCGGATGATATCGACCAGCTGCCGCAATACTTGAAAGATGCGTTCATGGTTTTGTACAA
CACCGTCAAAACGCTGGAAGAAGAACTGGAGCCGGAAGGCAACAGCTACCGTGGTTACTA
TGTTAAAGATGCGATGAAAGTTCTGGCGCGCGACTACTTCGTCGAGCACAAGTGGTATAAC
CGTCAGATTGTGCCGAGCGTCGAGGAATACCTGAAGATTAGCTGTATCAGCGTTGCCGTTC
ACATGGCAACGGTGCACTGCATCGCCGGTATGTACGAGATTGCGACGAAAGAAGCCTTCGA
ATGGTTGAAAACCGAGCCGAAGCTGGTTATCGACGCCAGCCTGATCGGTCGTTTGCTGGAC
GACATGCAAAGCACGAGCTTCGAGCAGCAGCGCGGCCATGTGAGCAGCGCTGTTCAGTGTT
ATATGGCGCAATATGGCGTGACCGCAGAAGAAGCGTGCGAGAAGCTGCGTGAGATGGCAG
CAATTGCGTGGAAAGATGTGAATGAAGCCTGTCTGCGTCCGACTGTGTTTCCGATGCCGAT
CCTGCTGCCGAGCATTAACCTGGCGCGTGTGGCAGAGGTCATCTATCTGCGTGGTGACGGT
TACACCCACGCGGGTGGCGAAACCAAGAAACATATCACCGCAATGCTGGTTAAGCCGATT
GAAGTGTAA
SEQ ID NO.: 10
   SCH51-998-28 from *D. winteri*, Open reading frame, wild type DNA sequence. 
ATGGATCTTAGTACTTCACCTGTTCTTTCTTCCTCCCCCTTCCGGTGGAAGACGGAAAAAA
TCCGGCCGTTCGCCGTTCAGCTGGATTTCACCCCAGTATTTGGGGTGATCATTTCCTCTCCT
ACACTGAAGATCACAAGAAGCTGGATGCATGGAGCGAAAGGACTCAAGTGTTGAAGGAAG
AGGTGAGGAGAATTTTAATCAATGCCAAGGGGTCACTAGAAGAGTTGGATTTGTTGGATGC
AATCCAACGCCTTGGGGTGAAATATCACTTTGAGAAAGAGATTGAAGAGGCATTACACCAT
ATTTATGTTGCAGAAACTCATGTTTCTACTGATGACTTATATTCCGTTTCTCTCCGGTTTCGA
CTTCTTAGACAACAAGGGTACAATGTATCTGCTGATGTATTTAAAAAGTTCAAAGATGAGA
GGGGCAACTTCAAGGCAAGCTTAAGTACTGATGCCAGGGGGTTGCTAAGCTTGTATGAAGC
TGCATTTCTCAGCATACGAGGAGATGATATCTTAGATGAAGCCATAACTTTCACAAGAGAG
CAGCTTAAGTCTTCTATGACCCATGTTGATGCCCCTCTTGCCAAACAAATAGCCCATGCCTT
AGAGGTACCAGCGCACAAGCGCATACAAAGACTAGAGAACATTCGCTACCTCACAATCTA
CCAAGAAGAGAAAGGAAGGAATGATGTGTTGCTTGAGCTTGCCAAGTTGGATTTCAATATC
 
TTACAACAATTGCATAAGAAAGAACTGAGAGACCTTACAAAGTGGTGGAAGGACACAGAC
GTTGCAGGAAAGCTACCTTTCATCAGAGATAGGTTGGTGGAATGCTATTATTGGATCTTGG
GTGTGTATTATGAGCCAGAATACTCCAGAGCTAGAATTTTTCTACCAAAATGACAATCAT
GGTCTCAGTTGTTGATGACATATATGACGTATATGCTACTGAAGATGAGCTCCAACTATTCA
CTGATGCAATCTATAGGTGGGATCTGGAGGGCCTAGATCAACTCCCACAGTTCTTGAAAGA
CTGTTTTCTTGTACTCTATGACACCGTCAAGGAATTAGAAGACGAACTAGAACCGGAAGGA
AAATCCTATCGTGGATACTATGTAAAGGATGCGATGAAGGTTTTGGCTAGAGATTACTTCG
TTGAGCACAAATGGTATAACAGAAACATAGTGCCAAGTGTAGAAGAATATCTCCGTGTTTC
TTGCATCAGTGTTGCAGTCCATATGGCTAACGTCCATTGCTGTGCTGGATGGAGATGTA
ATGAGCAAAGAGGCATTCGAATGGTTGAAGAGTGAACCAAAGGTTGTAATGGATGCATCA
CTAATTGGCCGACTGCTCGATGACATGCAGTCCACCGAGTTTGAGCAAAAGAGAGGCCATG
TTGCATCGGCTGTCCAATGTTACATGAATGAGTATGGAGTGACTTACAAAGAAGCGTGTGA
AAAGCTGCATGAAATGGCTGCCCTTGCATGGAAAGACGTAAACCAGGCTTGCCTTAAACCA
ACTGTTTTCCCTCTCCCTGTATTTATGCCTGCAATCAACCTTGCGCGAGTGGCTGAAGTCAT
CTACCTTCGTGGAGATGGGTATACTCATTCAGGAGGAGAGACTAAAGAAAATATCACGTTG
ATGCTTGTCAATCCAATCTCTGTGTGA
SEQ ID NO.: 11
   SCH51-998-28 from *D. winteri,* amino acid sequence. 
MDLSTSPVLSSSPLPVEDGKNPAVRRSAGFHPSIWGDHFLSYTEDHKKLDAWSERTQVLKEEV
RRILINAKGSLEELDLLDAIQRLGVKYHFEKEIEEALHHIYVAETHVSTDDLYSVSLRFRLLRQQ
GYNVSADVFKKFKDERGNFKASLSTDARGLLSLYEAAFLSIRGDDILDEAITFTREQLKSSMTH
VDAPLAKQIAHALEVPAHKRIQRLENIRYLTIYQEEKGRNDVLLELAKLDFNILQQLHKKELRD
LTKWWKDTDVAGKLPFIRDRLVECYYWILGVYYEPEYSRARIFSTKMTIMVSVVDDIYDVYAT
EDELQLFTDAIYRWDLEGLDQLPQFLKDCFLVLYDTVKELEDELEPEGKSYRGYYVKDAMKVL
ARDYFVEHKWYNRNIVPSVEEYLRVSCISVAVHMANVHCCAGMGDVMSKEAFEWLKSEPKV
VMDASLIGRLLDDMQSTEFEQKRGHVASAVQCYMNEYGVTYKEACEKLHEMAALAWKDVN
QACLKPTVFPLPVFMPAINLARVAEVIYLRGDGYTHSGGETKENITLMLVNPISV
SEQ ID NO.: 12
   SCH51-998-28_opt from *D. winteri,* Codon optimized DNA sequence of SCH51-998-28. 
ATGGATCTGAGCACCAGTCCGGTTCTGAGCAGCTCACCGCTGCCGGTTGAAGATGGTAAAA
ATCCGGCAGTTCGTCGTAGCGCAGGTTTTCATCCGAGCATTTGGGGTGATCATTTTCTGAGC
TATACCGAGGATCACAAAAACTGGATGCATGGTCAGAACGTACCCAGGTTCTGAAAGAA
GAAGTGCGTCGTATTCTGATTAATGCAAAAGGTAGCCTGGAAGAACTGGATCTGCTGGATG
CAATTCAGCGTCTGGGTGTTAAATATCACTTTGAGAAAGAAATCGAAGAAGCCCTGCATCA
TATTTATGTTGCAGAAACCCATGTGTCAACCGATGATCTGTATAGCGTTAGCCTGCGTTTTC
GTCTGCTGCGTCAGCAGGGTTATAATGTTAGCGCAGATGTGTTCAAAAAATTCAAAGATGA
ACGCGGTAACTTCAAAGCAAGCCTGAGCACCGATGCACGTGGTCTGCTGAGCCTGTATGAA
GCAGCATTTCTGAGCATTCGTGGTGATGATATTCTGGATGAAGCAATTACCTTTACCCGTGA
ACAGCTGAAAAGCAGCATGACCCATGTTGATGCACCGCTGGCAAAACAAATTGCACATGC
ACTGGAAGTTCCGGCACATAAACGTATTCAGCGCCTGGAAAATATTCGCTATCTGACCATT
TACCAAGAAGAGAAAGGTCGTAACGATGTTCTGCTGGAACTGGCCAAACTGGATTTTAACA
TTCTGCAGCAGCTGCATAAAAAAGAACTGCGTGATCTGACCAAATGGTGGAAAGATACCG
ATGTTGCAGGTAAACTGCCGTTTATTCGTGATCGTCTGGTTGAATGCTATTATTGGATTCTG
GGCGTTTATTATGAGCCGGAATATAGCCGTGCACGTATTTTTAGCACCAAAATGACCATTAT
GGTTAGCGTGGTGGATGACATCTATGATGTTTATGCAACCGAAGATGAACTGCAGCTGTTT
ACCGATGCAATTTATCGTTGGGATCTGGAAGGTCTGGATCAGCTGCCGCAGTTCCTGAAAG
ATTGTTTTCTGGTTCTGTATGATACCGTGAAAGAACTGGAAGATGAGCTGGAACCGGAAGG
TAAAAGCTATCGTGGTTATTATGTTAAAGATGCCATGAAAGTTCTGGCACGCGATTATTTTG
TTGAGCACAAATGGTATAACCGCAATATTGTTCCGAGCGTGGAAGAATATCTGCGTGTTAG
CTGTATTAGCGTTGCAGTTCACATGGCAAATGTTCATTGTTGTGCAGGTATGGGTGATGTGA
TGAGCAAAGAAGCATTTGAATGGCTGAAAAGTGAACCGAAAGTTGTTATGGATGCCAGCCT
GATTGGTCGCCTGCTGGACGATATGCAGAGCACCGAATTTGAACAGAAACGTGGTCATGTT
GCAAGCGCAGTTCAGTGTTATATGAATGAATATGGCGTGACCTATAAAGAGGCATGCGAAA
AACTGCATGAAATGGCAGCACTGGCATGGAAAGATGTTAATCAGGCATGTCTGAAACCGA
CCGTTTTTCCGCTGCCTGTTTTTATGCCTGCAATTAATCTGGCACGTGTTGCCGAAGTTATTT
ACCTGCGTGGGGATGGTTATACCCATAGCGGTGGTGAAACCAAAGAAAACATTACCCTGAT
GCTGGTTAATCCGATTAGCGTTTAA
SEQ ID NO.: 13
   SCH52-13163-6 from *D. lanceolata*, DNA sequence. 
ATGGATGTTCTAATTCCTCCCCTGTGGCTTCCACTCTCCCTCTGCCCGAAGATGGAAACTT
GGACGTCGTTCGCAGATCCGCCGGGTTTCATCCGACGGTCTGGGGCGATCACTTCCTCGCTT
ACTCGCCCGATCCAACCAAAATAGATGCTTGGACTAAAAGAGTTGAAGAGCTGAAGCAAG
AAGTGAAGAGGATTCTAAGCAATGTGAAAGGGTCACTGGAAGAGCTGAACTTGCTTGATG
CTATCCAACACCTTGGGATTGGTTATCATTTTGAGAAAGAGATTGATGATGCTTTACAACTA
ATCTTTGATTCCCATATTGATGCTTTTCCTACTGATGATCTATATGTGGCTGCCCTCCGATTT
AGCCTACTAAGGCGACAAGGGCACTGTGTTTCTTCAGATGTATTCAAAAAATTCAAAGATG
AGCAGGGGAATTTCAAGGCAGAGCTGAGCACCGATGCGAAAGGTTTGCTGAGTCTCTATGA
CGCGGCGTATCTCAGTGTAAGAGGGGAAGATATATTGGATGAGGCCATTCCTTTCACTAGG
GAGCACCTTAGGACTTGTATTAGCCATGTAGATTCTCATTTGGCAGCAAAAATTGAGCATTC
TCTAGAGCTTCCCCTGCATCATCGCATACCAAGGCTAGAGAACAGGCACTACATCTCAGTG
TACGAAGGAGAGAAGGAAAGGAATGAAGTTGTACTAGAGCTTGCCAAATTAGATTTCAAT
CTGATTCAAATCTTGCACCAAAGAGAGCTGAGGGACATCACAACGTGGTGGAATGAGATTG
ACCTCGCAGCAAAGCTACCATTTATTAGGGATAGGTTGGTGGAGTGCTACTATTGGATCAT
GGGTGTCTATTTTGAACCAATATTCTCAAGGGCTAGAGTTTTTCGACCAAAATGACAATTT
TGGTCTCAGTTGTCGACGACATATATGATGTCTACGCTACAGAGGATGAGCTCCAACTTTTC
ACTGACGCAATCTATAGGTGGGATGCCGAGGACATTGAGCAGCTTCCACAGTACTTGAAAG
ATTCTTTTCTTGTACTCTATAACACCGTGAAGGACTTAGAAGAGGAGCTGAAACCAGAAGG
AAACTCATATCGTGGAGACTATGTAAAAGATGCGATGAAGGTTTTGGCAAGAGATTACTTT
GTGGAGCACAAATGGTATAACAGAAAAATTGTACCGTCAGTAGAGGACTACCTACGAATTT
CTTGCATTAGTGTTGCCGTTCATATGGCTACAGTTCATTGTTGTGCTGGGATGGATGAAATT
GCAACCAAAGAGGCATTCGAATGGTTGAAGACCGAACCTAAACTTGTTATAGATGCATCAC
TGATTGGGCGTCTCCTCGATGACATGCAGTCCACCTCGTTTGAGCAACAGAGAGGTCATGT
GTCATCGGCGGTACAGTGTTACATGATCCAATATGGCGTATCACACGAAGAAGCGTGTGAG
AAGTTGACAGAAATGGCTGCAATTGCATGGAAAGATGTAAACCAAGCATGCCTTAGGCCC
ACTGTTTTCCCAATGCCTATTCTTCTGCCTTCAATCAACCTTGCACGTGTGGCAGAAGTCAT
CTACCTGCGCGGAGATGGATATACACATGCTGGTGGTGAGACCAAAAAACATATCACGGCC
ATGCTTGTTGAACCAATCCAAGTCTGA
SEQ ID NO.: 14
   SCH52-13163-6 from *D. lanceolata,* Open reading frame, wild type DNA sequence. 
MDVLIPSPVASTLPLPEDGNLDVVRRSAGFHPTVWGDHFLAYSPDPTKIDAWTKRVEELKQEV
KRILSNVKGSLEELNLLDAIQHLGIGYHFEKEIDDALQLIFDSHIDAFPTDDLYVAALRFSLLRRQ
GHCVSSDVFKKFKDEQGNFKAELSTDAKGLLSLYDAAYLSVRGEDILDEAIPFTREHLRTCISH
VDSHLAAKIEHSLELPLHHRIPRLENRHYISVYEGEKERNEVVLELAKLDFNLIQILHQRELRDIT
TWWNEIDLAAKLPFIRDRLVECYYWIMGVYFEPIFSRARVFSTKMTILVSVVDDIYDVYATEDE
LQLFTDAIYRWDAEDIEQLPQYLKDSFLVLYNTVKDLEEELKPEGNSYRGDYVKDAMKVLAR
DYFVEHKWYNRKIVPSVEDYLRISCISVAVHMATVHCCAGMDEIATKEAFEWLKTEPKLVIDA
SLIGRLLDDMQSTSFEQQRGHVSSAVQCYMIQYGVSHEEACEKLTEMAAIAWKDVNQACLRP
T VFPMPILLPSINLARVAEVIYLRGDGYTHAGGETKKHITAMLVEPIQV
SEQ ID NO.: 15
   SCH52-13163-6_opt, Codon optimized DNA sequence of SCH51-13163-6.

ATGGATGTTCTGATTCCGAGTCCGGTTGCAAGCACCCTGCCGCTGCCGGAAGATGGTAATC
TGGATGTTGTTCGTCGTAGCGCAGGTTTCATCCGACCGTTTGGGGTGATCATTTCTGGCA
TATAGTCCGGATCCGACCAAAATTGATGCATGGACCAAACGTGTTGAGGAACTGAAACAA
GAAGTGAAACGTATTCTGAGCAATGTGAAAGGTAGCCTGGAAGAACTGAATCTGCTGGAT
GCAATTCAGCATCTGGGTATTGGTTATCACTTCGAGAAAGAAATTGATGATGCACTGCAGC
TGATCTTTGATAGCCATATTGATGCCTTTCCGACCGATGATCTGTATGTTGCAGCACTGCGT
TTTAGCCTGCTGCGTCGTCAGGGTCATTGTGTTAGCAGTGATGTTTTCAAAAAATTCAAAGA
CGAGCAGGGCAACTTTAAAGCAGAACTGAGCACCGATGCAAAAGGTCTGCTGAGCCTGTA
TGATGCCGCATATCTGAGCGTTCGTGGTGAAGATATTCTGGATGAAGCAATTCCGTTTACCC
GTGAACATCTGCGTACCTGTATTAGCCATGTGGATAGCCATCTGGCAGCAAAAATTGAACA
TAGTCTGGAACTGCCTCTGCATCATCGTATTCCGCGTCTGGAAAATCGTCACTATATTAGCG
TTTATGAAGGCGAAAAAGAACGCAATGAAGTTGTGCTGGAACTGGCAAAACTGGATTTTAA
CCTGATTCAGATTCTGCATCAGCGTGAACTGCGTGATATTACCACCTGGTGGAATGAAATT
GACCTGGCAGCCAAACTGCCGTTTATTCGTGATCGTCTGGTTGAATGCTATTATTGGATTAT

GGGCGTGTATTTTGAACCGATTTTTAGCCGTGCACGTGTGTTTAGCACCAAAATGACCATTC
TGGTTAGCGTGGTGGATGATATCTATGATGTTTATGCAACCGAAGATGAGCTGCAACTGTTT
ACCGATGCCATTTATCGTTGGGATGCAGAAGATATTGAACAGCTGCCTCAGTATCTGAAAG
ATAGCTTTCTGGTTCTGTACAACACCGTGAAAGATCTGGAAGAAGAACTGAAACCGGAAGG
TAATAGCTATCGTGGTGATTATGTTAAAGACGCCATGAAAGTTCTGGCACGCGATTATTTTG
TTGAGCACAAATGGTATAACCGCAAAATTGTTCCGAGCGTGGAAGATTATCTGCGTATTAG
CTGCATTAGCGTTGCAGTTCACATGGCAACCGTTCATTGTTGCAGGTATGGATGAAATTG
CAACCAAAGAAGCATTTGAGTGGCTGAAAACCGAACCGAAACTGGTTATTGATGCAAGCCT
GATTGGTCGTCTGCTGGACGATATGCAGTCAACCAGCTTTGAACAGCAGCGTGGTCATGTT
AGCAGCGCAGTTCAGTGTTATATGATTCAGTATGGTGTTAGCCATGAAGAAGCATGCGAAA
AACTGACCGAAATGGCAGCAATTGCATGGAAAGATGTTAATCAGGCATGTCTGCGTCCGAC
CGTGTTTCCTATGCCGATTCTGCTGCCGAGCATTAATCTGGCACGTGTTGCCGAAGTTATCT
ATCTGCGTGGTGATGGTTATACCCATGCCGGTGGTGAAACCAAAAAACATATTACCGCAAT
GCTGGTAGAACCGATTCAGGTTTAA

## Claims

1. A method of producing drimenol comprising:
i) contacting farnesyl diphosphate (FPP) with a polypeptide having drimenol synthase activity and comprising an amino acid sequence having at least 70% sequence identity to a sequence of SEQ ID NO: 2 to produce the drimenol; and
ii) optionally isolating the drimenol.

2. The method as recited in claim 1 wherein the drimenol is isolated.

3. The method as recited in claim 1 or 2 wherein the drimenol is produced with greater than or equal to 30% selectivity.

4. The method as recited in any one of claims 1 to 3 comprising contacting the drimenol with at least one enzyme to produce a drimenol derivative.

5. The method as recited in any one of claims 1 to 3 comprising converting the drimenol to a drimenol derivative using a chemical synthesis.

6. An isolated polypeptide having drimenol synthase activity comprising an amino acid sequence having at least 70% sequence identity to an amino acid sequence of SEQ ID NO: 2.

7. An isolated nucleic acid molecule encoding a polypeptide recited in claim 6.

8. The nucleic acid molecule of claim 7 comprising the nucleic acid sequence of SEQ ID NO: 1 or SEQ ID NO: 3.

9. The method as recited in claim 1 comprising the steps of transforming a non-human host cell or non-human host organism with a nucleic acid encoding a polypeptide comprising an amino acid sequence having at least 70% sequence identity to a sequence of SEQ ID NO: 2, and culturing the host cell or host organism under conditions that allow for the production of the polypeptide.

10. A vector comprising the nucleic acid molecule of claims 7 or 8.

11. The vector of claim 10 wherein the vector is a prokaryotic vector, viral vector or a eukaryotic vector.

12. The vector of claim 10 or claim 11 that is an expression vector.

13. The method recited in claim 9 wherein the cell is a prokaryotic or eukaryotic cell.

14. The method as recited in claim 13 wherein the prokaryotic cell is a bacterial cell.

15. The method as recited in claim 13 wherein the eukaryotic cell is a yeast cell or a plant cell.

16. A non-human host organism or non-human host cell comprising the nucleic acid molecule of claim 7 or 8 or the vector of any one of claims 10 to 12.

17. The non-human host organism or non-human host cell of claim 16, wherein the non-human host organism or cell is a bacterium, a yeast, a fungal cell or a plant cell.

18. The non-human host organism or non-human host cell of claim 17, wherein the bacterium is *E. coli* and the yeast is *Saccharomyces cerevisiae.*

## Patentansprüche

1. Verfahren zur Herstellung von Drimenol, umfassend:
i) In-Kontakt-Bringen von Farnesyldiphosphat (FPP) mit einem Polypeptid, das Drimenol-Synthase-Aktivität aufweist und eine Aminosäuresequenz mit zumindest 70 % Sequenzidentität mit einer Sequenz von SEQ ID NO: 2 umfasst, zur Herstellung des Drimenols; und
ii) gegebenenfalls Isolieren des Drimenols.

2. Verfahren nach Anspruch 1, wobei das Drimenol isoliert wird.

3. Verfahren nach Anspruch 1 oder 2, wobei das Drimenol mit einer Selektivität größer als oder gleich 30 % hergestellt wird.

4. Verfahren nach einem der Ansprüche 1 bis 3, umfassend das In-Kontakt-Bringen des Drimenols mit zumindest einem Enzym, um ein Drimenol-Derivat herzustellen.

5. Verfahren nach einem der Ansprüche 1 bis 3, umfassend das Umwandeln des Drimenols zu einem Drimenol-Derivat mittels einer chemischen Synthese.

6. Isoliertes Polypeptid mit Drimenol-Synthase-Aktivität, umfassend eine Aminosäuresequenz, die zumindest 70 % Sequenzidentität mit einer Aminosäuresequenz von SEQ ID NO: 2 aufweist.

7. Isoliertes Nukleinsäuremolekül, codierend für ein Polypeptid nach Anspruch 6.

8. Nukleinsäuremolekül nach Anspruch 7, umfassend die Nukleinsäuresequenz von SEQ ID NO: 1 oder SEQ ID NO: 3.

9. Verfahren nach Anspruch 1, umfassend die Schritte des Transformierens einer nicht menschlichen Wirtszelle oder eines nicht menschlichen Wirtsorganismus mit einer Nukleinsäure, die für ein Polypeptid codiert, das eine Aminosäuresequenz umfasst, die zumindest 70 % Sequenzidentität mit einer Sequenz von SEQ ID NO: 2 aufweist, und des Kultivierens der Wirtszelle oder des Wirtsorganismus unter Bedingungen, die die Herstellung des Polypeptids zulassen.

10. Vektor, umfassend das Nukleinsäuremolekül nach Anspruch 7 oder 8.

11. Vektor nach Anspruch 10, wobei der Vektor ein prokaryotischer Vektor, ein viraler Vektor oder ein eukaryotischer Vektor ist.

12. Vektor nach Anspruch 10 oder Anspruch 11, der ein Expressionsvektor ist.

13. Verfahren nach Anspruch 9, wobei die Zelle eine prokaryotische oder eukaryotische Zelle ist.

14. Verfahren nach Anspruch 13, wobei die prokaryotische Zelle eine bakterielle Zelle ist.

15. Verfahren nach Anspruch 13, wobei die eukaryotische Zelle eine Hefezelle oder eine Pflanzenzelle ist.

16. Nicht menschlicher Wirtsorganismus oder nicht menschliche Wirtszelle, umfassend das Nukleinsäuremolekül nach Anspruch 7 oder 8 oder den Vektor nach einem der Ansprüche 10 bis 12.

17. Nicht menschlicher Wirtsorganismus oder nicht menschliche Wirtszelle nach Anspruch 16, wobei der nicht menschliche Wirtsorganismus oder die nicht menschliche Wirtszelle ein Bakterium, eine Hefe, eine Pilzzelle oder eine Pflanzenzelle ist.

18. Nicht menschlicher Wirtsorganismus oder nicht menschliche Wirtszelle nach Anspruch 17, wobei das Bakterium *E. coli* ist und die Hefe *Saccharomyces cerevisiae* ist.

## Revendications

1. Méthode pour la production de driménol comprenant:
i) la mise en contact de famésyldiphosphate (FPP) avec un polypeptide ayant une activité de driménol synthase et comprenant une séquence d'acides aminés possédant au moins 70% d'identité de séquence avec une séquence de la SEQ ID NO: 2 pour produire le driménol; et
ii) optionnellement l'isolement du driménol.

2. Méthode selon la revendication 1, dans laquelle le driménol est isolé.

3. Méthode selon les revendications 1 ou 2, dans laquelle le driménol est produit avec une sélectivité supérieure ou égale à 30%.

4. Méthode selon l'une quelconque des revendications 1 à 3, comprenant la mise en contact du driménol avec au moins une enzyme pour produire un dérivé de driménol.

5. Méthode selon l'une quelconque des revendications 1 à 3, comprenant la conversion du driménol en un dérivé de driménol en utilisant une synthèse chimique.

6. Polypeptide isolé ayant une activité de driménol synthase et comprenant une séquence d'acides aminés possédant au moins 70% d'identité de séquence avec une séquence d'acides aminés de la SEQ ID NO: 2.

7. Molécule d'acide nucléique isolée codant pour un polypeptide selon la revendication 6.

8. Molécule d'acide nucléique selon la revendication 7 comprenant la séquence d'acide nucléique de la SEQ ID NO: 1 ou la SEQ ID NO: 3.

9. Méthode selon la revendication 1 comprenant les étapes de transformation d'une cellule hôte non humaine ou d'un organisme hôte non humain avec un acide nucléique codant pour un polypeptide comprenant une séquence d'acides aminés possédant au moins 70% d'identité de séquence avec une séquence de la SEQ ID NO: 2 et de culture de la cellule hôte ou de l'organisme hôte dans des conditions qui permettent la production du polypeptide.

10. Vecteur comprenant la molécule d'acide nucléique selon les revendications 7 ou 8.

11. Vecteur selon la revendication 10, où le vecteur est un vecteur procaryote, un vecteur viral ou un vecteur eucaryote.

12. Vecteur selon la revendication 10 ou revendication 11 qui est un vecteur d'expression.

13. Méthode selon la revendication 9, dans laquelle la cellule est une cellule procaryote ou eucaryote.

14. Méthode selon la revendication 13, dans laquelle la cellule procaryote est une cellule bactérienne.

15. Méthode selon la revendication 13, dans laquelle la cellule eucaryote est une cellule de levure ou une cellule de plante.

16. Organisme hôte non humain ou cellule hôte non humaine comprenant la molécule d'acide nucléique selon les revendications 7 ou 8 ou le vecteur selon l'une quelconque des revendications 10 à 12.

17. Organisme hôte non humain ou cellule hôte non humaine selon la revendication 16, où l'organisme non humain ou la cellule hôte est une bactérie, une levure, une cellule fongique ou une cellule de plante.

18. Organisme hôte non humain ou cellule hôte non humaine selon la revendication 17, où la bactérie est E. coli et la levure est Saccharomyces cerevisiae.
